# EUROPEAN PATENT SPECIFICATION

(11) **EP 1 642 966 B1**
(45) Date of publication and mention of the grant of the patent: **03.03.2010**
(21) Application number: 04747086.9
(22) Date of filing: 30.06.2004
(51) Int. Cl.: C12N 7/01, C12N 15/45, C12N 15/86

(54) **MINUS STRAND RNA VIRAL VECTORS CARRYING A GENE WITH ALTERED HYPERMUTABLE REGIONS**
NEGATIV-STRANG RNA-VIRUS-VEKTOREN, WELCHE EIN GEN MIT VERÄNDERTEN HYPERMUTIERBAREN REGIONEN TRAGEN
VECTEURS VIRAUX DE TYPE ARN A POLARITE NEGATIVE COMPORTANT UN GENE AVEC DES REGIONS HYPERMUTABLES MODIFIEES

(30) Priority: 30.06.2003 JP 2003187312
(43) Date of publication of application: 05.04.2006
(73) Proprietor: Dnavec Research Inc., Tsukuba-shi, Ibaraki 305-0856 (JP)
(72) Inventor: YOU, Jun; c/o Dnavec Research Inc., Tsukuba-shi, Ibaraki 3050856 (JP); IIDA, Akihiro; c/o Dnavec Research Inc., Tsukuba-shi, Ibaraki 3050856 (JP); HASEGAWA, Mamoru; c/o Dnavec Research Inc., Tsukuba-shi, Ibaraki 3050856 (JP)
(74) Representative: Vossius & Partner
(86) International application number: PCT/JP2004/009617
(87) International publication number: WO 2005/001082

(56) References cited:
- JP-A- 2003 513 633
- HINZMAN EDWARD E ET AL: "Identification of an upstream sequence element required for vesicular stomatitis virus mRNA transcription" JOURNAL OF VIROLOGY, vol. 76, no. 15, August 2002 (2002-08), pages 7632-7641, XP002384348 ISSN: 0022-538X
- GUAN H ET AL: "Analysis of cis-Acting Sequences Involved in Plus-Strand Synthesis of a Turnip Crinkle Virus-Associated Satellite RNA Identifies a New Carmovirus Replication Element" VIROLOGY, ACADEMIC PRESS,ORLANDO, US, vol. 268, no. 2, 15 March 2000 (2000-03-15), pages 345-354, XP004436078 ISSN: 0042-6822

### SEQUENCE LISTING

<110> DNAVEC RESEARCH INC.
<120> MINUS STRAND RNA VIRUS VECTOR CARRING GENE MODIFIED IN HIGH MUTATION REGION
<130> D3-A0302P
<140>
   <141>
<150> JP 2003-187312
   <151> 2003-06-30
<160> 110
<170> PatentIn version 3.1
<210> 1
   <211> 10
   <212> DNA
   <213> Artificial
<220>
   <223> a mutagenic sequence for minus strand RNA viruses
<400> 1
   agaaaaacyy 10
<210> 2
   <211> 11
   <212> DNA
   <213> Artificial
<220>
   <223> a mutagenic sequence for minus strand RNA viruses
<400> 2
   agaaaaaacy y 11
<210> 3
   <211> 10
   <212> DNA
   <213> Artificial
<220>
   <223> a mutagenic sequence for minus strand RNA viruses
<400> 3
   agaaaaactt 10
<210> 4
   <211> 11
   <212> DNA
   <213> Artificial
<220>
   <223> a mutagenic sequence for minus strand RNA viruses
<400> 4
   agaaaaaact t 11
<210> 5
   <211> 12
   <212> DNA
   <213> Artificial
<220>
   <223> an example of E sequence of Sendai virus
<220>
   <221> misc_feature
   <222> (2).. (2)
   <223> "n" at location 2 stands for any of a, g, c, or t
<400> 5
   antaagaaaa ac 12
<210> 6
   <211> 10
   <212> RNA
   <213> Artificial
<220>
   <223> an example of S sequence of Sendai virus
<400> 6
   cwuuvwcccu 10
<210> 7
   <211> 10
   <212> RNA
   <213> Artificial
<220>
   <223> an example of S sequence of Sendai virus
<400> 7
   cuuugacccu 10
<210> 8
   <211> 10
   <212> RNA
   <213> Artificial
<220>
   <223> an example of S sequence of Sendai virus
<400> 8
   cauucacccu 10
<210> 9
   <211> 10
   <212> RNA
   <213> Artificial
<220>
   <223> an example of S sequence of Sendai virus
<400> 9
   cuuucacccu 10
<210> 10
   <211> 10
   <212> DNA
   <213> Artificial
<220>
   <223> an example of S sequence of Sendai virus
<400> 10
   agggtcaaag 10
<210> 11
   <211> 10
   <212> DNA
   <213> Artificial
<220>
   <223> an example of S sequence of Sendai virus
<400> 11
   agggtgaatg 10
<210> 12
   <211> 10
   <212> DNA
   <213> Artificial
<220>
   <223> an example of S sequence of Sendai virus
<400> 12
   agggtgaaag 10
<210> 13
   <211> 9
   <212> RNA
   <213> Artificial
<220>
   <223> an example of E sequence of Sendai virus
<400> 13
   uuuuucuua 9
<210> 14
   <211> 9
   <212> DNA
   <213> Artificial
<220>
   <223> an example of E sequence of Sendai virus
<400> 14
   taagaaaaa 9
<210> 15
   <211> 10
   <212> DNA
   <213> Artificial
<220>
   <223> an example of S sequence of Sendai virus
<400> 15
   ctttcaccct 10
<210> 16
   <211> 15
   <212> DNA
   <213> Artificial
<220>
   <223> an example of E sequence of Sendai virus
<400> 16
   tttttcttac tacgg 15
<210> 17
   <211> 18
   <212> DNA
   <213> Artificial
<220>
   <223> an artificially synthesized linker sequence
<400> 17
   atgcatgccg gcagatga 18
<210> 18
   <211> 18
   <212> DNA
   <213> Artificial
<220>
   <223> an artificially synthesized primer sequence
<400> 18
   gttgagtact gcaagagc 18
<210> 19
   <211> 42
   <212> DNA
   <213> Artificial
<220>
   <223> an artificially synthesized primer sequence
<400> 19
   tttgccggca tgcatgtttc ccaaggggag agttttgcaa cc 42
<210> 20
   <211> 18
   <212> DNA
   <213> Artificial
<220>
   <223> an artificially synthesized primer sequence
<400> 20
   atgcatgccg gcagatga 18
<210> 21
   <211> 21
   <212> DNA
   <213> Artificial
<220>
   <223> an artificially synthesized primer sequence
<400> 21
   tgggtgaatg agagaatcag c 21
<210> 22
   <211> 51
   <212> DNA
   <213> Artificial
<220>
   <223> an artificially synthesized primer sequence
<400> 22
   acttgcggcc gccaaagttc aatgcagagg tcgcctctgg aaaaggccag c 51
<210> 23
   <211> 76
   <212> DNA
   <213> Artificial
<220>
   <223> an artificially synthesized primer sequence
<400> 23
   atccgcggcc gcgatgaact ttcaccctaa gtttttctta ctacggctaa agccttgtat 60
   cttgcacctc ttcttc 76
<210> 24
   <211> 24
   <212> DNA
   <213> Artificial
<220>
   <223> an artificially synthesized primer sequence
<400> 24
   tcacgcggcc gccaaagttc aatg 24
<210> 25
   <211> 24
   <212> DNA
   <213> Artificial
<220>
   <223> an artificially synthesized primer sequence
<400> 25
   atctgcggcc gcgatgaact ttca 24
<210> 26
   <211> 24
   <212> DNA
   <213> Artificial
<220>
   <223> an artificially synthesized primer sequence
<400> 26
   taacaatagg aagacctcta atgg 24
<210> 27
   <211> 24
   <212> DNA
   <213> Artificial
<220>
   <223> an artificially synthesized primer sequence
<400> 27
   ccattagagg tcttcctatt gtta 24
<210> 28
   <211> 24
   <212> DNA
   <213> Artificial
<220>
   <223> an artificially synthesized primer sequence
<400> 28
   aacatttagg aagaatttgg atcc 24
<210> 29
   <211> 24
   <212> DNA
   <213> Artificial
<220>
   <223> an artificially synthesized primer sequence
<400> 29
   ggatccaaat tcttcctaaa tgtt 24
<210> 30
   <211> 22
   <212> DNA
   <213> Artificial
<220>
   <223> an artificially synthesized primer sequence
<400> 30
   cggtgaggag gactgttcga gc 22
<210> 31
   <211> 20
   <212> DNA
   <213> Artificial
<220>
   <223> an artificially synthesized primer sequence
<400> 31
   cagttcagtc aagtttgcct 20
<210> 32
   <211> 19
   <212> DNA
   <213> Artificial
<220>
   <223> an artificially synthesized primer sequence
<400> 32
   cgaccaattt agtgcagaa 19
<210> 33
   <211> 21
   <212> DNA
   <213> Artificial
<220>
   <223> an artificially synthesized primer sequence
<400> 33
   ttcccttcat cgactatgac c 21
<210> 34
   <211> 24
   <212> DNA
   <213> Artificial
<220>
   <223> an artificially synthesized primer sequence
<400> 34
   atgcagaggt cgcctctgga aaag 24
<210> 35
   <211> 21
   <212> DNA
   <213> Artificial
<220>
   <223> an artificially synthesized primer sequence
<400> 35
   cacattggaa tgcagatgag a 21
<210> 36
   <211> 20
   <212> DNA
   <213> Artificial
<220>
   <223> an artificially synthesized primer sequence
<400> 36
   tatctgtgct tccctatgca 20
<210> 37
   <211> 19
   <212> DNA
   <213> Artificial
<220>
   <223> an artificially synthesized primer sequence
<400> 37
   gcacagtgga agaatttca 19
<210> 38
   <211> 19
   <212> DNA
   <213> Artificial
<220>
   <223> an artificially synthesized primer sequence
<400> 38
   ggagtgcctt tttgatgat 19
<210> 39
   <211> 20
   <212> DNA
   <213> Artificial
<220>
   <223> an artificially synthesized primer sequence
<400> 39
   ggatgacctt ctgcctctta 20
<210> 40
   <211> 21
   <212> DNA
   <213> Artificial
<220>
   <223> an artificially synthesized primer sequence
<400> 40
   ggatagcttg atgcgatctg t 21
<210> 41
   <211> 20
   <212> DNA
   <213> Artificial
<220>
   <223> an artificially synthesized primer sequence
<400> 41
   ggaaagttgc agatgaggtt 20
<210> 42
   <211> 24
   <212> DNA
   <213> Artificial
<220>
   <223> an artificially synthesized primer sequence
<400> 42
   ctaaagcctt gtatcttgca cctc 24
<210> 43
   <211> 20
   <212> DNA
   <213> Artificial
<220>
   <223> an artificially synthesized primer sequence
<400> 43
   acctcatctg caactttcca 20
<210> 44
   <211> 19
   <212> DNA
   <213> Artificial
<220>
   <223> an artificially synthesized primer sequence
<400> 44
   catggctaaa gtcaggata 19
<210> 45
   <211> 19
   <212> DNA
   <213> Artificial
<220>
   <223> an artificially synthesized primer sequence
<400> 45
   tctattaaga atcccacct 19
<210> 46
   <211> 20
   <212> DNA
   <213> Artificial
<220>
   <223> an artificially synthesized primer sequence
<400> 46
   gtctggctgt agattttgga 20
<210> 47
   <211> 21
   <212> DNA
   <213> Artificial
<220>
   <223> an artificially synthesized primer sequence
<400> 47
   tgaagtcttg cctgctccag t 21
<210> 48
   <211> 24
   <212> DNA
   <213> Artificial
<220>
   <223> an artificially synthesized primer sequence
<400> 48
   agtatctcac ataggctgcc ttcc 24
<210> 49
   <211> 20
   <212> DNA
   <213> Artificial
<220>
   <223> an artificially synthesized primer sequence
<400> 49
   ggagcagtgt cctcacaata 20
<210> 50
   <211> 14
   <212> DNA
   <213> Homo sapiens
<400> 50
   caatagaaaa actt 14
<210> 51
   <211> 14
   <212> DNA
   <213> Homo sapiens
<400> 51
   caataggaaa actt 14
<210> 52
   <211> 15
   <212> DNA
   <213> Homo sapiens
<400> 52
   atttagaaaa aactt 15
<210> 53
   <211> 15
   <212> DNA
   <213> Homo sapiens
<400> 53
   atttaggaga acctt 15
<210> 54
   <211> 14
   <212> DNA
   <213> Sendai virus
<400> 54
   agtaagaaaa actt 14
<210> 55
   <211> 14
   <212> DNA
   <213> Sendai virus
<400> 55
   attaagaaaa actt 14
<210> 56
   <211> 14
   <212> DNA
   <213> Sendai virus
<400> 56
   aataagaaaa actt 14
<210> 57
   <211> 14
   <212> DNA
   <213> Artificial
<220>
   <223> an altered human CFTR gene (region around the position 1257)
<400> 57
   caataggaag acgt 14
<210> 58
   <211> 15
   <212> DNA
   <213> Artificial
<220>
   <223> an altered human CFTR gene (region around the position 3905)
<400> 58
   atttaggaag aattt 15
<210> 59
   <211> 11
   <212> RNA
   <213> Artificial
<220>
   <223> an example of E sequence
<220>
   <221> misc_feature
   <222> (10).. (10)
   <223> "n" at location 10 stands for any of a, g, c, or u
<400> 59
   uuuuucuuan u 11
<210> 60
   <211> 11
   <212> RNA
   <213> Artificial
<220>
   <223> an example of E sequence
<400> 60
   uuuuubwyww u 11
<210> 61
   <211> 11
   <212> RNA
   <213> Artificial
<220>
   <223> an example of E sequence
<400> 61
   uuuuucuuau u 11
<210> 62
   <211> 11
   <212> RNA
   <213> Artificial
<220>
   <223> an example of E sequence
<400> 62
   uuuuugauua u 11
<210> 63
   <211> 11
   <212> RNA
   <213> Artificial
<220>
   <223> an example of E sequence
<400> 63
   uuuuuuauaa u 11
<210> 64
   <211> 11
   <212> RNA
   <213> Artificial
<220>
   <223> an example of E sequence
<400> 64
   uuuuuuauau u 11
<210> 65
   <211> 11
   <212> RNA
   <213> Artificial
<220>
   <223> an example of E sequence
<400> 65
   uuuuugucua u 11
<210> 66
   <211> 11
   <212> RNA
   <213> Artificial
<220>
   <223> an example of E sequence
<220>
   <221> misc_feature
   <222> (5) .. (5)
   <223> "n" at location 5 stands for any of a, g, c, or u
<400> 66
   uuuunhwuar y 11
<210> 67
   <211> 11
   <212> RNA
   <213> Artificial
<220>
   <223> an example of E sequence
<400> 67
   uuuuuuauaa c 11
<210> 68
   <211> 11
   <212> RNA
   <213> Artificial
<220>
   <223> an example of E sequence
<400> 68
   uuuuguuuag u 11
<210> 69
   <211> 11
   <212> RNA
   <213> Artificial
<220>
   <223> an example of E sequence
<400> 69
   uuuuaauuaa c 11
<210> 70
   <211> 11
   <212> RNA
   <213> Artificial
<220>
   <223> an example of E sequence
<400> 70
   uuuuucuuaa u 11
<210> 71
   <211> 11
   <212> RNA
   <213> Artificial
<220>
   <223> an example of E sequence
<400> 71
   uuuucuuuaa u 11
<210> 72
   <211> 10
   <212> RNA
   <213> Artificial
<220>
   <223> an example of E sequence
<400> 72
   uuuuuykaaw 10
<210> 73
   <211> 11
   <212> RNA
   <213> Artificial
<220>
   <223> an example of E sequence
<400> 73
   uuuuuuguaa a 11
<210> 74
   <211> 10
   <212> RNA
   <213> Artificial
<220>
   <223> an example of E sequence
<400> 74
   uuuuwwwkwa 10
<210> 75
   <211> 10
   <212> RNA
   <213> Artificial
<220>
   <223> an example of E sequence
<400> 75
   uuuuauauua 10
<210> 76
   <211> 10
   <212> RNA
   <213> Artificial
<220>
   <223> an example of E sequence
<400> 76
   uuuuaaauua 10
<210> 77
   <211> 10
   <212> RNA
   <213> Artificial
<220>
   <223> an example of E sequence
<400> 77
   uuuuuuauua 10
<210> 78
   <211> 10
   <212> RNA
   <213> Artificial
<220>
   <223> an example of E sequence
<400> 78
   uuuuuuugua 10
<210> 79
   <211> 10
   <212> RNA
   <213> Artificial
<220>
   <223> an example of E sequence
<400> 79
   uuuuuaauua 10
<210> 80
   <211> 10
   <212> RNA
   <213> Artificial
<220>
   <223> an example of E sequence
<400> 80
   uuuuuaagua 10
<210> 81
   <211> 10
   <212> RNA
   <213> Artificial
<220>
   <223> an example of E sequence
<400> 81
   uuuuauauaa 10
<210> 82
   <211> 10
   <212> RNA
   <213> Artificial
<220>
   <223> an example of E sequence
<400> 82
   uuuuaaauaa 10
<210> 83
   <211> 10
   <212> RNA
   <213> Artificial
<220>
   <223> an example of E sequence
<400> 83
   uuuuuaauaa 10
<210> 84
   <211> 10
   <212> RNA
   <213> Artificial
<220>
   <223> an example of E sequence
<400> 84
   uuuucuwwra 10
<210> 85
   <211> 11
   <212> RNA
   <213> Artificial
<220>
   <223> an example of E sequence
<400> 85
   uuuuucuwwr a 11
<210> 86
   <211> 12
   <212> RNA
   <213> Artificial
<220>
   <223> an example of E sequence
<400> 86
   uuuuuucuww ra 12
<210> 87
   <211> 13
   <212> RNA
   <213> Artificial
<220>
   <223> an example of E sequence
<400> 87
   uuuuuuucuw wra 13
<210> 88
   <211> 10
   <212> RNA
   <213> Artificial
<220>
   <223> an example of E sequence
<400> 88
   uuuucuauga 10
<210> 89
   <211> 11
   <212> RNA
   <213> Artificial
<220>
   <223> an example of E sequence
<400> 89
   uuuuucuaug a 11
<210> 90
   <211> 12
   <212> RNA
   <213> Artificial
<220>
   <223> an example of E sequence
<400> 90
   uuuuuucuau ga 12
<210> 91
   <211> 13
   <212> RNA
   <213> Artificial
<220>
   <223> an example of E sequence
<400> 91
   uuuuuuucua uga 13
<210> 92
   <211> 10
   <212> RNA
   <213> Artificial
<220>
   <223> an example of E sequence
<400> 92
   uuuucuuaaa 10
<210> 93
   <211> 11
   <212> RNA
   <213> Artificial
<220>
   <223> an example of E sequence
<400> 93
   uuuuucuuaa a 11
<210> 94
   <211> 12
   <212> RNA
   <213> Artificial
<220>
   <223> an example of E sequence
<400> 94
   uuuuuucuua aa 12
<210> 95
   <211> 13
   <212> RNA
   <213> Artificial
<220>
   <223> an example of E sequence
<400> 95
   uuuuuuucuu aaa 13
<210> 96
   <211> 10
   <212> RNA
   <213> Artificial
<220>
   <223> an example of E sequence
<400> 96
   uuuuuucuwa 10
<210> 97
   <211> 11
   <212> RNA
   <213> Artificial
<220>
   <223> an example of E sequence
<400> 97
   uuuuuuucau a 11
<210> 98
   <211> 13
   <212> RNA
   <213> Artificial
<220>
   <223> an example of E sequence
<400> 98
   uuuuuuucwh rwy 13
<210> 99
   <211> 13
   <212> RNA
   <213> Artificial
<220>
   <223> an example of E sequence
<400> 99
   uuuuuuucau gau 13
<210> 100
   <211> 13
   <212> RNA
   <213> Artificial
<220>
   <223> an example of E sequence
<400> 100
   uuuuuuucau guu 13
<210> 101
   <211> 13
   <212> RNA
   <213> Artificial
<220>
   <223> an example of E sequence
<400> 101
   uuuuuuucac auc 13
<210> 102
   <211> 13
   <212> RNA
   <213> Artificial
<220>
   <223> an example of E sequence
<400> 102
   uuuuuuucuc gac 13
<210> 103
   <211> 13
   <212> RNA
   <213> Artificial
<220>
   <223> an example of E sequence
<400> 103
   uuuuuuucaa gau 13
<210> 104
   <211> 13
   <212> RNA
   <213> Artificial
<220>
   <223> an example of E sequence
<400> 104
   uuuuuuusur ucu 13
<210> 105
   <211> 13
   <212> RNA
   <213> Artificial
<220>
   <223> an example of E sequence
<400> 105
   uuuuuuucua ucu 13
<210> 106
   <211> 13
   <212> RNA
   <213> Artificial
<220>
   <223> an example of E sequence
<400> 106
   uuuuuuucug ucu 13
<210> 107
   <211> 13
   <212> RNA
   <213> Artificial
<220>
   <223> an example of E sequence
<400> 107
   uuuuuuugua ucu 13
<210> 108
   <211> 13
   <212> RNA
   <213> Artificial
<220>
   <223> an example of E sequence
<400> 108
   uuuuuuucwa ucu 13
<210> 109
   <211> 13
   <212> RNA
   <213> Artificial
<220>
   <223> an example of E sequence
<400> 109
   uuuuuuucur ucu 13
<210> 110
   <211> 11
   <212> RNA
   <213> Artificial
<220>
   <223> an example of E sequence
<400> 110
   uuuuucuuam u 11

## Description

### Technical Field

The present invention relates to minus-strand RNA viruses carrying a gen whose hypermutable region(s) has been altered.

### Background Art

Minus-strand RNA viruses are viruses that have their viral genes encoded in RNA genomes (minus strand RNAs) in an antisense fashion. When a minus-strand RNA virus infects cells, antigenome RNA (plus strand) is generated from the ROTA genome (minus strand) which serves as a template, and then from the antigenome RNA, genome RNA is generated and packaged into viral particles to allow virus propagation. In recent years, development of gene introduction via vectors has taken advantage of the ability of minus-strand RNA viruses to express genes at high levels in the cytoplasm of infected host cells (International Patent Publication Nos. WO00/70055 and WO00/70070). However, little is known regarding the high frequency occurrence of mutations when certain foreign genes are inserted into a minus-strand RNA viral vector. In addition, no means of vector alteration are available for preventing such mutations.

### Disclosure of the Invention

An objective of the present invention is to provide a Sendai virus carrying a human CFTR gene whose hypermutable region(s) has been altered. A further object of the invention is to provide a pharmaceutical composition comprising said virus, and the use of said pharmaceutical composition for gene therapy.

In previous studies using Sendai virus (SeV), almost no nucleotide mutations have been found in viruses carrying foreign genes even after continuous passages over many generations. It is therefore understood that, in general, the genome stability of minus-strand RNA virus is high and foreign genes that have been inserted are stably expressed for a long period (Yu, D. et al., Genes Cells 2, 457-466 (1997)). The present inventors constructed SeV vectors to express the human CFTR gene (hCFTR), which is the causative gene of cystic fibrosis (CF), and discovered that the SeV vector carrying wild-type hCFTR produced a mutated hCFTR gene with high frequency and that the normal protein was no longer expressed.

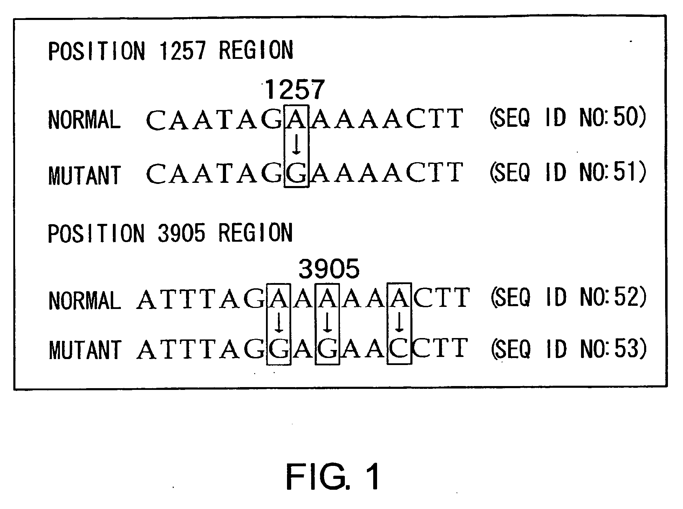

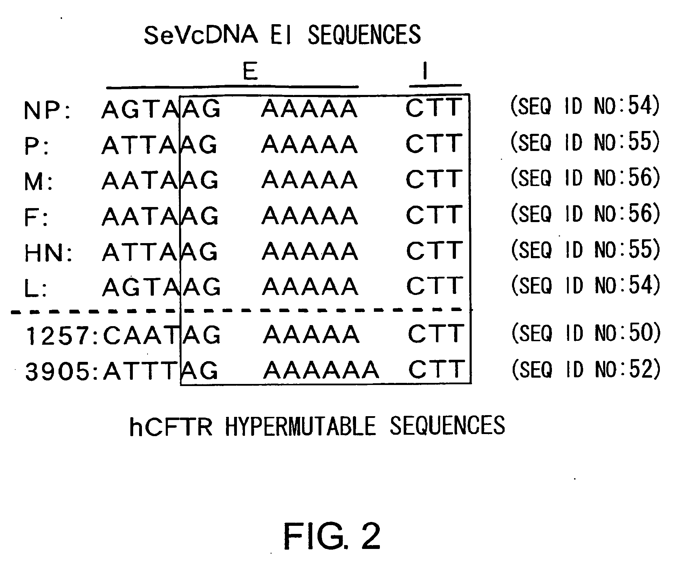

The inventors analyzed the hCFTR gene sequence in the virus genome of the SeV vector carrying the full-length hCFTR gene in detail, and then discovered that nucleotide mutations were generated with high frequency in two regions (Fig. 1). These nucleotide mutations altered the amino acid sequence of the encoded protein, resulting in loss of hCFTR protein activity. The present inventors suspected that these two hypermutable regions are mutation hot spots because they comprise sequences with relatively high homology to the EI (End-Intergenic) sequence of minus-strand RNA viruses (Fig. 2). To reduce the EI sequence homology, the nucleotides sequences in these two regions were artificially altered without changing their amino acid sequences. The altered gene was inserted into a SeV vector. The results showed no mutations of the hCFTR gene in the genome when the SeV vector is equipped with the human CFTR gene whose hypermutable regions have been altered. Furthermore, the vector-introduced cells stably expressed the normal hCFTR protein both *in vitro* and *in vivo*.

As described above, the present inventors identified sequences that undergo mutations with high frequency in a foreign gene when the gene is carried by minus-strand RNA viruses, and succeeded in reducing the mutation frequency by altering these sequences. Minus-strand RNA virus is promising as a gene therapy vector. For such applications, it is important that genes carried by the viral vector are stable and undergo no mutations. The present invention enables the provision of minus-strand RNA viral vectors carrying a gene whose stability has been improved.

Specifically, the present invention relates to minus-strand RNA viruses carrying a gene with an altered hypermutable region(s) and methods for producing such viruses. Specifically, the present invention provides the following items:
(1) A Sendai virus carrying a human CFTR gene, wherein the gene has been altered at the 5'-AGAAAAAC-3' and 5'-AGAAAAAAC-3' sequence comprised in the sense strand of the wild type of a human CFTR gene in such a way that the amino acid sequence encoded by the 5'-AGAAAAAC-3' and 5'-AGAAAAAAC-3' sequence of the wild-type gene is maintained.
(2) A DNA encoding the genome RNA of the Sendai virus of item (1) or a complementary strand thereof.
(3) The Sendai virus of item (1), wherein the 5'-AGAAAAAC-3' and 5'-AGAAAAAAC-3' sequence is part of a 5'-AGAAAAACTT-3' and 5'-AGAAAAAACTT-3' sequence.
(4) A pharmaceutical composition comprising the virus of item (1) or (3).
(5) Use of the virus according to item (1) or (3) for the preparation of a pharmaceutical composition for gene therapy.

Also disclosed herein is a method for producing a minus-strand RNA virus carrying a gene which is altered to lower mutation frequency, wherein the gene before alteration comprises in its sense strand sequence a part of an antigenome E sequence of the minus-strand RNA virus, and wherein the method comprises the steps of:
(a) altering the part of the E sequence in the sense strand sequence to a different sequence to lower identity to the E sequence;
(b) preparing a DNA encoding the genome of the minus-strand RNA virus into which the altered gene has been inserted, or a complementary strand thereof; and
(c) reconstituting the minus-strand RNA virus by transcribing the DNA. Further disclosed

herein is a method for producing a minus-strand RNA virus carrying a gene which is altered to lower mutation frequency, wherein the gene sequence before alteration comprises a 5'-AGAAAAAC-3' or 5'-AGAAAAAAC-3' sequence, and wherein the method comprises the steps of:
(a) altering the 5'-AGAAAAAC-3' or 5'-AGAAAAAAC-3' sequence;
(b) preparing a DNA encoding the genome of the minus-strand RNA virus into which the altered gene has been inserted, or a complementary strand thereof; and
(c) reconstituting the minus-strand DNA virus by transcribing the DNA. In the latter method, the 5'-AGAAAAAC-3' or 5'-AGAAAAAAC-3' sequence may be part of a 5'-AGAAAAACTT-3' or 5'-AGAAAAAACTT-3' sequence; and
the gene may be a human CFTR gene. In both methods described before, the minus-strand RNA virus may be a paramyxovirus; and more specifically the minus-strand RNA virus may be a Sendai virus.

The present invention provides a Sendai virus carrying a human CFTR gene with altered hypermutable regions. Specifically, the human CFTR gene has been altered at the 5'-AGAAAAAC-3' and 5'-AGAAAAAAC-3' sequence comprised in the sense strand of the wild type of a human CFTR gene in such a way that the amino acid sequence encoded by the 5'-AGAAAAAC-3' and 5'-AGAAAAAAC-3' sequence of the wild type gene is maintained. The present inventors discovered that mutations are generated with high frequency in a foreign gene when the foreign gene comprises a partial E sequence of minus-strand RNA virus and is carried by a minus-strand RNA virus. Alteration of this sequence to a different sequence significantly suppress the generation of mutations. Hence, when preparing a recombinant minus-strand RNA virus carrying a gene comprising the sequence, the inserted gene can be prevented from undergoing mutations by inserting the gene into the virus after the sequence has been altered. The recombinant virus refers to a virus generated from a recombinant polynucleotide, namely, a polynucleotide resulting from artificial alterations (breakage or ligation) of polynucleotide chain linkages. Recombinant polynucleotides do not have their two ends linked in the same way as naturally occurring polynucleotides. Recombinant polynucleotides can be produced by conventional genetic engineering technologies combining polynucleotide synthesis, nuclease treatment, ligase treatment, and the like. Recombinant viruses can be produced through virus reconstitution by expressing polynucleotides encoding a virus genome that has been constructed by genetic engineering (Molecular Cloning: A Laboratory Manual (2000) 3rd ed., Sambrook, J. and DW Russel, Cold Spring Harbor Laboratory Press).

Herein, a "gene" refers to a genetic material, specifically a nucleic acid encoding a transcriptional unit(s). For the foreign gene to be inserted into a virus of the present invention, the original sequence before alteration (for example, the sequence of a wild-type gene) is not limited as long has the gene comprises a partial E sequence of the minus-strand RNA. virus carrying the gene, and includes those that may and may not encode a protein. Herein, a protein-encoding nucleic acid is also referred to as a "proteins gene". Likewise, a ribozyme-encodins nucleic acid is referred to as a "ribozyme gene". For example, when a sequence such as 5'-AGAAAAAC-3' or 5'-AGAAAAAAC-3' which comprises a portion of a Sendai virus E sequence (i.e., a sequence comprising a portion of an antigenome strand (plus strand) E sequence) is used, a virus of the present invention would have a modified foreign gene, in which the sequence 5'-AGAAAAAC-3' (corresponding to 3'-UCUUUUUG-5' on the minus-strand viral genome sequence) or 5'-AGAAAAAAC-3' (corresponding to 3'-UCUUUUUUG-5' on the minus-strand viral genome sequence) of the wild-type gene sense strand has been altered to a different sequences. In other words 3'-UCUUUUUG-5'or 3'-CUUUUUUG-5', which is a complementary sequence of the 5'-AGAAAAAC-3' or 5'-AGAAAAAAC-3' sequence, is altered in the foreign gene on minus-strand RNA virus genomic RNA. Since a minus-strand RNA virus is a virus having minus-strand (antisense strand) genome, the viral genes are encoded as antisense sequences. Accordingly, when a "wild-type gene comprising a 5'-AGAAAAAC-3' or 5'-AGAAAAAAC-3' sequence without sequence modification is inserted into a minus-strand RNA viral vector, the 3'-UCUUUUUG-5'or 3'-CUUUUUUG-5' sequence becomes comprised in the gene sequence carried by the minus-strand genome of the vector (i.e., comprised in the antisense strand sequence of the gene because the genome is a minus strand). However, the 3'-UCUUUUUG-5'or 3'-CUUUUUUG-5' sequence has been altered for the viruses of the present invention.

Accordingly, with respect to the recombinant Sendai virus carrying a human CFTR gene provided by the present invention, when the wild type human CFTR gene comprising the 5'-AGAAAAAC-3' and 5'-AGAAAAAAC-3' sequence without sequence modification is inserted into the Sendai viral vector, the 3'-UCUUUUUG-5' and 3'-UCUUUUUUG-5' sequence becomes comprised in the gene sequence carried by the minus-strand genome of the vector (i.e., comprised in the antisense strand sequence of the gene because the genome is a minus strand).

Herein, the minus-strand RNA virus refers to an RNA virus having minus-strand (negative-strand) genome. The minus-strand RNA virus includes, for example, Sendai virus, Newcastle disease virus, mumps virus, measles virus, RS virus (respiratory syncytial virus), rinderpest virus, and distemper virus, which belong to the Paramyxoviridae family; influenza virus belonging to the Orthomyxoviridae family; and vesicular stomatitis virus and rabies virus belonging to the Rhabdoviridae family. The particularly preferred viruses are those having single-stranded minus-strand genome (mononega viruses), including, for example, viruses belonging to the families of Paramyxoviridae (including the genera *Respirovirus, Morbillivirus, Rubulavirus,* and *Pneumovirus),* Rhabdoviridae (including the genera *Vesiculovirus*, *Lyssavirus*, and *Ephemerovirus*), Filoviridae, Orthomyxoviridae (including influenza viruses A, B, and C, and Thogoto-like viruses), Bunyaviridae (including the genera *Bunyavirus, Hantavirus, Nairovirus,* and *Phlebovirus),* and Arenaviridae.

A paramyxovirus refers to a virus belonging to Paramyxoviridae, or to derivatives thereof. Paramyxoviruses are a group of viruses with non-segmented negative strand RNA as their genome, and they include Paramyxovirinae (including *Respirovirus* (also referred to as *Parampovirus*), *Rubulavirus,* and *Morbillivirus*), and Pneumovirinae (including *Pneumovirus* and *Metapneumovirus).* Specific examples of *Paramyxovirus* applicable to the present invention are Sendai virus, Newcastle disease virus, mumps virus, measles virus, respiratory syncytial virus (RS virus), rinderpest virus, distemper virus, simian parainfluenza virus (SV5), and human parainfluenza viruses 1, 2, and 3. Viruses disclosed herein are preferably those belonging to Paramyxovirinae (including *Respirovirus*, disclosed herein are preferably those belonging to Paramyxovirinae (including *Respirovirus, Rubulavirus,* and *Morbillivirus*) or derivatives thereof, and more preferably those belonging to the genus *Respirovirus* (also referred to as *Paramyxovirus*) or derivatives thereof. Examples of viruses of the genus *Respirovirus* applicable to this invention are human parainfluenza virus-1 (HPIV-1), human parainfluenza virus-3 (HPIV-3), bovine parainfluenza virus-3 (BPIV-3), Sendai virus (also referred to as murine parainfluenza virus-1), and simian parainfluenza virus-10 (SPIV-10). The most preferred paramyxovirus disclosed herein is Sendai virus. These viruses may be derived from natural strains, wild strains, mutant strains, laboratory-passaged strains, artificially constructed strains, or the like.

A "vector" also disclosed herein is a carrier for introducing a nucleic acid into a cell. Minus-strand RNA viral vectors are carriers derived from minus-strand RNA viruses to introduce nucleic acids into cells. Minus-strand RNA viruses such as SeV described above are excellent gene transfer vectors. Since paramyxoviruses carry out transcription and replication only in the cytoplasm of host cells, and since they don't have a DNA phase, chromosomal integration does not occur. Therefore, they do not give rise to safety problems caused by chromosomal abberations, such as canceration or immortalization. This characteristic of the minus-strand RNA virus contributes a great deal to safety when using a paramyxovirus as a vector. This virus has further qualitative merits, such as flexibility in the size of the genes to be inserted and in the packaging thereof, since it does not have a capsid structure protein. For example, transmissible SeV vector can introduce a foreign gene of at least 4 kb in size, and can simultaneously express two or more genes by adding transcription units.

Wild type SeV is known to be pathogenic to rodents, causing pneumonia; however, it is not pathogenic to humans. This was supported by a previous report that nasal administration of wild type SeV to non-human primates does not show severe adverse effects (Hurwitz, J. L. et al., Vaccine 15: 533-540, 1997). The two points below, "high infectivity" and "high expression level", should also be noted as advantages. SeV vectors infect cells by binding to sialic acids in the sugar chains of cell membrane proteins. This sialic acid is expressed in almost all cells, giving rise to a broad infection spectrum, *i.e.,* high infectivity. When a transmissible SeV replicon-based vector releases viruses, these viruses re-infect neighboring cells, replicating multiple ribonucleoprotein (RNP) copies in the cytoplasm of infected cells, and distributing these into daughter cells in line with cell division, and therefore continuous expression can be expected. Further, SeV vectors can be applied to an extremely wide range of tissues. Furthermore, their characteristic expression mechanism, wherein transcription and replication occurs only in the cytoplasm, has been shown to express inserted genes at very high levels (Moriya, C. et al., FEBS Lett. 425(1) 105-111 (1998); WO00/70070). Furthermore, SeV vectors made non-transmissible by deleting an envelope gene have been successfully recovered (WO00/70070; Li, H.-O. et al., J. Virol. 74(14) 6564-6569 (2000)). Thus, SeV vectors have been improved to further enhance their "safety", while maintaining their "high infectivity" and "high expression levels".

These characteristics support the effectivity of minus-strand RNA viruses for gene therapy and gene transfer, and the likelihood that SeV will become a promising choice in gene therapy. By inserting a gene for treatment (or analysis) into a minus-strand RNA virus, and administering the vector locally, the therapeutic gene can be locally expressed at high levels, and definite therapeutic effects can be expected, along with reduced side effects. These effects are thought to be stronger for those minus-strand RNA viruses, including SeV, that can induce strong transient expression of inserted genes.

Minus-strand RNA viruses comprise minus-strand RNA viral genomic RNAs. A genomic RNA refers to an RNA that comprises the function of forming an RIVP with a viral protein of a minus-strand RNA virus, such that a gene in the genome is expressed by the protein, and that nucleic acid is replicated to form daughter RNPs. Minus-strand RNA viruses are viruses with a negative chain RNA in their genome, and such RNAs encode genes as antisense sequences. In general, in the minus-strand RNA viral genome, viral genes are arranged as antisense sequences between the 3'-leader region and the 5'-trailer region. Between the ORFs of respective genes are a transcription ending sequence (E sequence) - intervening sequence (I sequence) - transcription starting sequence (S sequence), such that each gene is transcribed as an individual cistron. Genomic RNAs in a virus of this invention comprise the antisense RNA sequences encoding N (nucleocapsid)-, P (phospho)-, and L (large)-proteins, which are viral proteins essential for the expression of the group of genes encoded by an RNA, and for the autonomous replication of the RNA itself. The RNAs may also encode M (matrix) proteins, essential for virion formation. Further, the RNAs may encode envelope proteins essential for virion infection. Minus-strand RNA viral envelope proteins include F (fusion) protein that causes cell membrane fusion, and HN (hemagglutinin-neuraminidase) protein, essential for viral adhesion to cells. However, HN protein is not required for the infection of certain types of cells (Markwell, M.A. et al., Proc. Natl. Acad. Sci. USA 82(4): 978-982 (1985)), and infection is achieved with F protein only. The RNAs may encode envelope proteins other than F protein and/or HN protein.

Minus-strand RNA viruses disclosed herein may be, for example, complexes of minus-strand RNA viral genomic RNAs and viral proteins, that is, ribonucleoproteins (RNPs). RNPs can be introduced into cells, for example, in combination with desired transfection reagents. Specifically, such RNPs are complexes comprising a minus-strand DNA viral genomic RNA, N protein, P protein, and L protein. On introducing an RNP into cells, cistrons encoding the viral proteins are transcribed from the genomic RNA by the action of viral proteins, and, at the same time, the genome itself is replicated to form daughter RNPs. Replication of a genomic RNA can be confirmed by using RT-PCR, Northern blot hybridization, or the like to detect an increase in the copy number of the RNA.

Further, minus-strand RNA viruses disclosed herein are preferably minus-strand RNA viral virions. "Virion" means a microparticle comprising a nucleic acid released from a cell by the action of viral proteins. Minus-strand RNA viral virions comprise structures in which an above-described RNP, comprising genomic RNA and viral proteins, is enclosed in a lipid membrane (referred to as an envelope), derived from the cell membrane. Virions may have infectivity. Infectivity refers to the ability of a minus-strand RNA virus to introduce nucleic acids in the virus into cells to which the virion has adhered, since they retain cell adhesion and membrane-fusion abilities. Minus-strand ROTA viruses of this invention may be transmissible or nontransmissible deficient viruses. "Transmissible" means that when a virus is introduced into a host cell, the virus can replicate itself within the cell to produce infectious virions.

For example, each gene in each virus belonging to Paramyxovirinae is generally described as below. In general, N gene is also described as "NP". Hemagglutinin with no neuraminidase activity is referred to as "H".

| | | | | | | | |
|---|---|---|---|---|---|---|---|
| *Respirovirus* | NP | P/C/V | M | F | HN | - | L |
| *Rubulavirus* | NP | P/V | M | F | HN | (SH) | L |
| *Morbillivirus* | NP | P/C/V | M | F | H | - | L |

For example, the database accession numbers for the nucleotide sequences of each of the Sendai virus genes are: M29343, M30202, M30203, M30204, M51331, M5565, M69046, and X17218 for N gene; M30202, M30203, M30204, M55565, M69046, X00583, X17007, and X17008 for P gene; D11446, K02742, M30202, M30203, M30204, M69046, U31956, X00584, and X53056 for M gene; D00152, D11446, D17334, D17335, M30202, M30203, M30204, M69046, X00152, and X02131 for F gene; D26475, M12397, M30202, M30203, M30204, M69046, X00586, X02808, and X56131 for HN gene; and D00053, M30202, M30203, M30204, M69040, X00587, and X58886 for L gene. Viral genes encoded by other viruses include, for example, N genes of CDV (AF014953), DMV (X73961), HPIV-1 (D01070), HPIV-2 (m55320), HPIV-3 (D10025), Mapuera (X85128), Mumps (D86172), MV (K01711), NDV (AF064091), PDPR (X74443), PDV (X76717), RPV (X68311), SeV (X00087), SV5 (M81442), and Tupaia (AF079780); P genes of CDV (X51869), DMV (Z47758), HPIV 1 (M74081), HPIV-3 (X04721), HPIV-4a (MS5975), HPIV-4b (M55976), Mumps (D86173); MV (M89920), NDV (M20302), PDV (X75960), RPV (X68311), SeV (M30202), SV5 (AF052755), and Tupaia (AF079780); C genes of CDV (AF014953), DMV (Z47758), HPIV-1 (M74081), HPIV (D00047), MV (AB016162), RPV (X68311), SeV (AB005796), and Tupaia (AF079780); M genes of CDV (M12669), DMV (Z30087), HPIV (S38067), HPIV-2 (M62734), HPIV-3 (D00130), HPIV-4a (D10241), HPIV-4b (D10242), Mumps (D86171), MV (AB012948), NDV (AF089819), PDPR (Z47977), PDV (X75717), RPV (M34018), SeV (U31956), and SV5 (M32248); F genes of CDV (M21849), DMV (AJ224704), HPN-1 (M22347), HPIV-2 (M60182), HPIV-3 (X05303), HPIV-4a (D49821), HPIV-4b (D49822), Mumps (D86169), MV (AB003178), NDV (AF048763), PDPR (Z37017), PDV (AJ224706), RPV (M21514), SeV (D17334), and SV5 (AB021962); and HN (H or G) genes of CDV (AF112189), DMV (AJ224705), HPIV (U709498), HPIV-2 (D000865), HPIV-3 (AB012132), HPIV-4A (M34033), HPIV-4B (AB006954), Mumps (X99040), MV (K01711), NDV (AF204872), PDPR (Z81358), PDV (Z36979), RPV (AF132934), SeV (U06433), and SV-5 (S76876). There are several strains known for each of the respective viruses, and thus some strains may have genes comprising sequences different from those exemplified above.

The ORFs of these viral proteins are arranged as antisense sequences in the genomic RNAs, via the above-described E-I-S (End-Intergenic-Start) sequence. The ORF closest to the 3'-end of the genomic RNAs only requires an S sequence between the 3'-leader region and the ORF, and does not require an E or I sequence. Further, the ORF closest to the 5'-end of the genomic RNA only requires an E sequence between the 5'-trailer region and the ORF, and does not require an I or S sequence. Furthermore, two ORFs can be transcribed as a single cistron, for example, by using an internal ribosome entry site (IRES) sequence. In such a case, an E-I-S sequence is not required between these two ORFs. In wild type paramyxoviruses, a typical RNA genome comprises a 3'-leader region, six ORFs encoding the N, P, M, F, HN (or H), and L proteins in the antisense and in this order, and a 5'-trailer region on the other end. In the genomic RNAs of viruses of this invention, as for the wild type viruses, it is preferable that ORFs encoding the N, P, M, F, HN (or H), and L proteins are arranged in this order, after the 3'-leader region, and before the 5'-trailer region; however, the gene arrangement is not limited to this. Certain types of minus-strand RNA virus do not comprise all six of these viral genes, but even in such cases, it is preferable to arrange each gene as in the wild type, as described above. In general, viruses maintaining the N, P, and L genes can autonomously express genes from the RNA genome in cells, replicating the genomic RNA. Furthermore, by the action of genes such as the F and HN (or H) genes, which encode envelope proteins, and the M gene, infectious virions are formed and released to the outside of cells. Thus, such viruses become transmissible viral vectors. A foreman gene may be inserted into a protein-noncoding region or the like in this genome, as described below.

Further, a minus-strand ROTA virus disclosed herein may be deficient in any of the wild type viral genes. For example, a minus-strand RNA virus that is inactivated in or deficient in the M, F, or HN gene, or any combinations thereof, can be a preferred virus of this invention. Such viruses can be reconstituted, for example, by externally supplying the products of the deficient genes. The viruses thus prepared adhere to host cells to cause cell fusion, as for wild type viruses, but they cannot form daughter virions that comprise the same infectivity as the original vector, because the viral genome introduced into cells is deficient in a viral gene. Therefore, such vectors are useful as safe viral vectors that can only introduce genes once. Examples of genes that the genome may be defective in are the F gene and/or HN (or H) gene. For example, recombinant viruses can be reconstituted by transecting host cells with a plasmid expressing a recombinant minus-strand RNA viral genome defective in the F gene, along with an F protein expression vector and expression vectors for the NP, P, and L proteins (WO00/0053 and WO00/70070; Li, H.-O. et al., J. Viol. 74(14) 6564-6569 (2000)). Viruses can also be produced by, for example, using host cells that have incorporated the F gene into their chromosomes. When supplying these proteins externally, their amino acid sequences do not need to be the same as the viral sequences, and a mutant or homologous gene from another virus may be used as a substitute, as long as their activity in nucleic acid introduction is the same as, or greater than, that of the natural type.

Further, recombinant viruses that comprise an envelope protein other than that of the virus from which the viral genome was derived, may be prepared as viruses of this invention. For example, when reconstituting a virus, a recombinant virus comprising a desired envelope protein can be generated by expressing an envelope protein other than the original envelope protein encoded by the basic viral genome. Such proteins are not particularly limited, and include the envelope proteins of other viruses, for example, the G protein of vesicular stomatitis virus (VSV-G) . The virus disclosed herein include pseudotype viruses comprising envelope proteins, such as VSV-G, derived from viruses other than the virus from which the genome was derived. By designing the viral vectors such that these envelope proteins are not encoded in RNA genomes, the proteins will never be expressed after virion infection of the cells.

Furthermore, a virus disclosed herein may be, for example, a vector with, on the envelope surface, a protein that can attach to a specific cell, such as an adhesion factor, ligand, receptor, antibody, or fragment thereof; or a vector comprising a chimeric protein with such a protein in the extracellular domain, and a polypeptide derived from the virus envelope in the intracellular domain. Thus, viral vectors that target specific tissues can also be produced. Such proteins may be encoded by the viral genome, or supplied by expressing genes other than the viral genome at the time of viral reconstitution (for example, other expression vectors or genes existing on host chromosomes).

Further, in the viruses of this invention, any viral gene comprised in the virus may be modified from the wild type gene in order to reduce the immunogenicity caused by viral proteins, or to enhance RNA transcriptional or replicational efficiency, for example. Specifically, for example, in a minus-strand RNA virus, modifying at least one of the N, P, and L genes, which are replication factors, is considered to enhance transcriptional or replicational function. Further, HN protein, which is an envelope protein, comprises both hemagglutinin activity and neuraminidase activity; however, it is possible, for example, to improve viral stability.in the blood if the former activity can be attenuated, and infectivity can be controlled if the latter activity is modified. Further, it is also possible to control membrane fusion ability by modifying F protein. For example, the epitopes of the F protein or HN protein, which can be cell surface antigenic molecules, can be analyzed, and using this, recombinant viruses with reduced antigenicity to these proteins can be prepared.

Furthermore, viruses of this invention may be deficient in accessory genes. For example, by knocking out the V gene, one of the SeV accessory genes, the pathogenicity of SeV toward hosts such as mice is remarkably reduced, without hindering gene expression and replication in cultured cells (Kato, A. et al., 1997, J. Virol. 71: 7266-7272; Kato, A. et al., 1997, EMBO J. 16: 578-587; Curran, J. et al., WO01/04272, EP1057179). Such attenuated viruses are particularly useful as less-toxic viral vectors for *in vivo* or *ex vivo* gene transfer.

Viruses disclosed herein encode an altered foreign gene in the minus-strand RNA viral genome described above (since the genome is a minus strand, the inserted gene is encoded as an antisense sequence). In the example of a foreign gene comprising a 5'-AGAAAAAC-3' or 5'-AGAAAAAAC-3' sequence, the 5'-AGAAAAAC-3' or 5'-AGAAAAAAC-3' sequence is comprised in the foreign gene sequence before alteration (for example, a wild-type gene) that corresponds to the region of the gene sequence carried by the virus. In contrast, the sequence is altered in the foreign gene carried by the viruses of the present invention. Specifically, the complementary sequence of 5'-AGAAAAAC-3' or 5'-AGAAAAAAC-3', 5'-GUUUUUCU-3' or 5'-GUUUUUUCU-3', is altered to a different sequence in a foreign gene carried by the minus-strand virus genome. The "wild-type gene" refers to a gene in naturally occurring organisms with a normal phenotype and includes any arbitrary polymorphisms. Such genes also include genes encoding an antibody directed against an antigen. As long as the foreign gene to be carried by a virus comprises a region corresponding to a wild type gene 5'-AGAAAAAC-3' or 5'-AGAAAAAAC-3' sequence, which has been altered before insertion into a vector, the other regions need not be identical to those on the wild-type gene. Specifically, the foreign gene carried by a virus may comprise an altered sequence(s) resulting from nucleotide addition, deletion, and/or substitution of the wild-type gene sequence in region other than the 5'-AGAAAAAC-3' or 5'-AGAAAAAAC-3' region. For example, when a nucleotide sequence encoding a transmembrane receptor extracellular domain comprises 5'-AGAAAAAC-3' or 5'-AGAAAAAAC-3', the virus carrying a foreign gene encoding only the extracellular domain, whose sequence has been altered, is included in the present invention. In addiction, when a nucleotide sequence encoding an antibody variable region comprises 5'-AGAAAAAC-3' or 5'-AGAAAAAAC-3', the virus encoding a synthetic protein, such as a Fab fragment or scFv, whose sequence has been altered is also included in the present invention. Furthermore, when a mutant gene comprises the 5'-AGAAAAAC-3' or 5'-AGAAAAAAC-3' sequence in a region corresponding to the 5'-AGAAAAAC-3' or 5'-AGAAAAAAC-3' sequence of a wild-type gene like the wild-type gene, the virus carrying the gene in which the 5'-AGAAAAAC-3' or 5'-AGAAAAAAC-3' sequence of the mutant gene has been altered is included in the viruses of the present invention. The above are illustrated using the 5'-AGAAAAAC-3' or 5'-AGAAAAAAC-3' sequence as an example of a sequence comprising a portion of an E sequence. Similarly, when a gene comprises any other portion of a minus-strand RNA virus E sequence, the rest of the regions may be natural sequences or altered sequences, as long as the gene comprises a sequence that is identical to the portion.

In a foreign gene carried by a minus-strand RNA viral vector of the present invention, the part of the foreign gene's wild-type sequence (sense strand) that is identical to a portion of an antigenome (plus strand) E sequence of the minus-strand RNA virus is substituted by a different sequence. Specifically, a portion of a minus-strand RNA virus E sequence refers to a partial sequence of the minus-strand RNA virus E sequence comprising 6 or more consecutive nucleotides, more specifically 7 or more consecutive nucleotides, still more specifically 8 or more consecutive nucleotides in the minus-strand RNA virus E sequence. More specifically, it is a partial sequence of a minus-strand RNA virus E sequence comprising 6 or more consecutive nucleotides, more specifically 7 or more consecutive nucleotides, still more specifically 8 or more consecutive nucleotides comprising an E sequence terminus of a minus-strand RNA virus. When a DNA sequence is compared with an RNA sequence, T (thymine) and U (uracil) are regarded as identical. The terminal nucleotide of a genome strand (minus strand) E sequence corresponds to U at the 5' end of the oligo U stretch at each viral gene's transcription germination site in the genome. The E sequence mentioned above refers to the E sequence of an arbitrary viral protein gene maintained in nature by a virus belonging to the same species as the minus-strand RNA virus carrying the foreign gene. Specifically, in one preferred embodiment, the foreign gene carried by a minus-strand RNA viral vector has been altered not to comprise any of the 6 or more consecutive nucleotides, 7 or more consecutive nucleotides, or 8 or more consecutive nucleotides at the E sequence termini.

Minus-strand RNA virus E sequences are well known. For example, Sendai virus E sequences include 3'-UNAUUCUUUUU-5' (N is any one of A, G, U, and C) (SEQ ID NO: 59; see Fig. 2), and human parainfluenza virus type 3 (HPIV-3) E sequences include 3'-UWWYWBUUUUU-5' (SEQ ID NO: 60), which specifically includes 3'-UUAUUCUUUUU-5' (SEQ ID NO: 61), 3'-UAUUAGUUUUU-5' (SEQ ID NO: 62), 3'-UAAUAUUUUUU-5' (SEQ ID NO: 63), 3'-UUAUAUUUUUU-5' (SEQ ID NO: 64), and 3'-UAUCUGUUUUU-5' (SEQ ID NO: 65) (Genbank accession X03967, M13886, M13888, and M21649). Measles virus E sequences includes YRAUWHNUUUU (SEQ ID NO: 66), which specifically includes 3'-CAAUAUUUUUU-5' (SEQ ID NO: 67), 3'-UAAUAUUUUUU-5' (SEQ ID NO: 63), 3'-UGAUUUGUUUU-5' (SEQ ID NO: 68), 3'-CAAUUAAUUUU-5' (SEQ ID NO: 69), 3'-UAAUUCUUUUU-5' (SEQ ID NO: 70), and 3'-UAAUUUCUUUU-5' (SEQ ID NO: 71) (Genbank accession K01711). Hendravirus E sequences include 3'-WAAKYUUUUU-5' (SEQ ID NO: 72), which specifically includes 3'-UAAUUCUUUUU-5' (SEQ ID NO: 70) and 3'=AAAUGUUUUUU-3' (SEQ ID NO: 73). Nipahvirus E sequences include 3'-UAAUUCUUUUU-5' (SEQ ID NO: 70; Genbank accession AF017149 and AF212302). The E sequence of RSV includes 3'-AWKWWWUUUU-5' (SEQ ID NO: 74), which specifically includes 3'-AUUAUAUUUU-5' (SEQ ID NO: 75), 3'-AUUAAAUUUU-5' (SEQ ID NO: 76), 3'-AUUAUUUUUU-5' (SEQ ID NO: 77), 3'-AUGUUUUUUU-5' (SEQ ID NO: 78), 3'-AUUAAUUUUU-5' (SEQ ID NO: 79), 3'-AUGAAUUUUU-5' (SEQ ID NO: 80), 3'-AAUAUAUUUU-5' (SEQ ID NO: 81), 3'-AAUAAAUUUU-5' (SEQ ID NO: 82), and 3'-AAUAAUUUUU-5' (SEQ ID NO: 83) (Genbank accession M74568). SV5 E sequences include 3'-ARWWUCU₄₋₇-5' (SEQ ID NOs: 84, 85, 86, and 87), which specifically includes 3'-AGUAUCU₄₋₇-5' (SEQ ID NOs: 88, 89, 90, and 91), 3'-AAAUUCU₄₋₇-5' (SEQ ID NOs: 92, 93, 94, and 95), and NDV E sequences include 3'-AWUCUUUUUU-5' (SEQ ID NO: 96) (Kuo Let al., J Virol. 1996, 70(10): 6892-901; Kuo L et al., J Virol. 1996, 70(9): 6143-50).

Among the viruses that belong to the Rhabdoviridae family, vesicular stomatitis virus comprises 3'-AUACUUUUUUU-5' (SEQ ID NO: 97; Genbank accession J02428) as an E sequence, and rabies virus comprises as an E sequence, 3'-YWRHWCUUUUUUU-5' (SEQ ID NO: 98) which specifically includes 3'-UAGUACUUUUUUU-5' (SEQ ID NO: 99), 3'-UUGUACUUUUUUU-5' (SEQ ID NO: 100), 3'-CUACACUUUUUUU-5' (SEQ ID NO: 101), 3'-CAGCUCUUUUUUU-5' (SEQ ID NO: 102), and 3'-UAGAACUUUUUUU-5' (SEQ ID NO: 103) (Genbank accession M31046). Among novirhabdoviruses, salmon infectious hematopoietic necrosis virus (IHNV) comprises as an E sequence, 3'-UCURUSUUUUUUU-5' (SEQ ID NO: 104) which specifically includes 3'-UCUAUCUUUUUUU-5' (SEQ ID NO: 105), 3'-UCUGUCUUUUUUU-5' (SEQ ID NO: 106), and 3'-UCUAUGUUUUUUU-5' (SEQ ID NO: 107) (Genbank accession L40883); viral haemorrhagic septicaemia virus (VHSV) comprises 3'-UCUAWCUUUUUUU-5' (SEQ ID NO: 108) (Genbank accession Y18263) as an E sequence; and hirame rahbdovirus and snakehead rhabdovirus (SHRV) comprise 3'-UCURUCUUUUUUU-5' (SEQ ID NO: 109; Genbank accession AF147498; Schnell MJ et al., J Virol. 1996, 70(4):2318-23; Barr JN et al., J Virol. 1997, 71(11):8718-25; Barr JN et al., J Virol: 1997, 71(3):1794-801; Tordo.N et al., Proc Natl Acad Sci U S A. 1986, 83(11):3914-8; Tordo N et al., Virology. 1988, 165(2):565-76) as an E sequence. Furthermore, the E sequences of viruses belonging to the Filoviridae family such as marburgvirus and ebolavirus, include 3'-UMAUUCUUUUU-5' (SEQ ID NO: 110; Genbank accession Z12132 and AF086833; Kiley MP et al., Virology. 1986, 149(2):251-4). In the above sequences, R is A or G; Y is C, T, or U; W is A, T, or U; S is G or C; M is A or C; K is G, T, or U; D is A, G, T, or U; H is A, C, T, or U; V is A, C, or G; B is C, G, T, or U; and N is A, C, G, T, or U. The above E sequences are shown as sequences in a viral genome (minus strand). The terminal nucleotide of each E sequence corresponds to the 5' terminal nucleotide of the sequence.

When compared with the E sequence of a minus-strand RNA virus used to carry a foreign gene, if the antisense strand sequence of the foreign gene is found to comprise a sequence identical to any 6, 7, or 8 consecutive nucleotides of the E sequence, or in a more specific embodiment, a sequence identical to 6, 7, or 8 consecutive nucleotides comprising, a terminus of the E sequence, generation of mutations in the foreign gene carried by the minus-strand RNA viral vector can be suppressed by altering the sequence. The phrase "altering a sequence" means altering a sequence to a distinct sequence by deleting some nucleotides from the sequence, substituting some nucleotides in the sequence with different nucleotides, or adding some nucleotides, or through a desired combination of the treatments. When the sequence to be altered is located in the coding region of a gene, the sequence is typically altered by substituting with different nucleotides. The alteration reduces the level of identity to the E sequence and thereby suppresses the introduction of mutations into the gene carried by the minus-strand RNA viral vector. Specifically, the probability or rate of generation of mutations can be reduced compared with the case where the sequence is not altered.

The subject of alteration is a desired sequence comprising a sequence identical to a portion of the E sequence of a minus-strand RNA virus. In particular, a desired sequence comprising 5'-AGAAAAAC-3' or 5'-AGAAAAAAC-3' in its sense sequence is a target of alteration. More specifically, such a sequence comprises 5'-AGAAAAAC[C/T][C/T]-3' or 5'-AGAAAAAAC[C/T][C/T]-3' ([C/T] = C or T) (SEQ ID NO: 1 or 2) in its sense strand, and still more specifically 5'-AGAAAAACTT-3' or 5'-AGAAAAAACTT-3' (SEQ ID NO: 3 or 4). A partial E sequence of the minus-strand RNA virus to which a foreign gene to be inserted is also a preferred target of alteration. Such a foreign gene comprises a partial sequence of 11 consecutive nucleotides or less, more specifically 10. consecutive nucleotide or less, still more specifically 9 consecutive nucleotides or less, yet more specifically 8 consecutive nucleotides or less of a minus-strand RNA virus E sequence. For example, sequences not identical to 5'-ANTAAGAAAAAC-3' (N= A, G, C, or T) (SEQ ID NO: 5) which comprises a complete SeV E sequence can also be targets of alteration.

Sequences to be modified when altering a partial E sequence in a foreign gene are not particularly limited. For example, such an altered sequence is obtained by appropriately adding, substituting, and/or deleting nucleotides while maintaining the gene function. For example, when this sequence is a portion of a sequence encoding a protein, it is preferable to alter the nucleotide sequence but not the amino acid sequence of the encoded protein. In general, a nucleotide sequence can be easily altered while maintaining the amino acid sequence by applying codon redundancy. For example, AGA (Arg) can be replaced with AGG or CGN (N= A, G, T, or C). AAA (Lys) can be replaced with AAG. AAC (Asn) can be replaced with AAT. ACT (Thr) can be replaced with ACA, ACC, or ACG. For example, the ORF of human CFTR gene contains two units of 5'-AGAAAAAC-3' or 5'-AGAAAAAAC-3' (Accession Nos. M28668, NM_000492; Riordan, J.R. et al., Science 245: 1066-1073 (1989)). Generation of mutations in the gene carried by a minus-strand RNA, virus can be prevented by altering these two sequences. The 5'-AGAAAAAC-3' or 5'-AGAAAAAAC-3' sequence may be deleted or replaced with another sequence if it does not encode a protein, as long as the deletion does not result in functional deficiency of the gene. For example, a sequence that forms base pairs in RNA secondary structure or the like is altered while maintaining the base pair formation. The sequence alteration can be achieved, for example, by PCR using synthetic oligonucleotides (see Examples).

Also disclosed herein are methods for producing viruses of the present invention. When a gene to be carried by a minus-strand RNA virus comprises in its sense strand sequence before alteration a portion of an antigenome E sequence of the minus-strand RNA virus, a method for producing viruses of the present invention comprises the steps of: (a) altering the partial E sequence in the sense strand sequence of the gene to decrease the level of identity to the E sequence; (b) preparing a DNA encoding the genome of the minus-strand RNA virus into which the altered gene has been inserted, or a complementary strand thereof; and (c) reconstituting the minus-strand RNA virus through transcription of the DNA. A minus-strand RNA, virus carrying a gene which has been modified to decrease mutation frequency can be prepared by the method described above. In one embodiment, when the 5'-AGAAAAAC-3' or 5'-AGAAAAAAC-3' sequence is comprised in the gene to be carried by a virus before alteration, a method for producing viruses of the present invention comprises the steps of: (a) altering the 5'-AGAAAAAC-3' or 5'-AGAAAAAAC-3' sequence; (b) preparing a DNA encoding the genome of the minus-strand RNA virus into which the altered gene has been inserted, or the complementary strand thereof; and (c) reconstituting the minus-strand RNA virus through transcription of the DNA.

A nucleic acid encoding a foreign gene can be inserted at any desired position in a protein-noncoding region in the genome, for example. The above nucleic acid can be inserted, for example, between the 3'-leader region and the viral protein ORF closest to the 3'-end; between each of the viral protein ORFs; and/or between the viral protein ORF closest to the 5'-end and the 5'-trailer region. Further, in genomes deficient in the F or HN (or H) gene or the like, nucleic acids encoding foreign genes can be inserted into those deficient regions. When introducing a foreign gene into a paramyxovirus, it is desirable to insert the gene such that the chain length of the polynucleotide to be inserted into the genome will be a multiple of six (Kolakofski, D. et al., J. Virol. 72:891-899, 1998; Calain, P. and Roux, L., J. Viol. 67:4822-4830, 1993; Calain, P. and Roux, L., J. Viol. 67: 4822-4830, 1993). An E-I-S sequence should be arranged between the inserted foreign gene and the viral protein ORF. Two or more sequences encoding foreign genes can be inserted in tandem via E-I-S sequences. Alternatively, a desired gene may be inserted though an IRES (internal ribosome entry site).

Expression levels of a foreign gene carried in a virus can be controlled using the type of transcriptional initiation sequence added upstream (to the 3'-side of the minus strand (the negative strand)) of the gene (WO01/18223). The expression levels can also be controlled of the position at which the foreign gene is inserted in the genome: the nearer to the 3'-end of the minus strand the insertion position is, the higher the expression level; while the nearer to the 5'-end the insertion position is, the lower the expression level. Thus, to obtain a desired gene expression level, the insertion position of a foreign gene can be appropriately controlled such that the combination with genes encoding the viral proteins before and after the foreign gene is most suitable. In general, since a high expression level of the foreign gene is thought to be advantageous, it is preferable to link a foreign gene to a highly efficient transcriptional initiation sequence, and to insert it near the 3'-end of the minus strand genome. Specifically, a foreign gene is inserted between the 3'-leader region and the viral protein ORF closest to the 3'-end. Alternatively, a foreign gene may be inserted between the ORFs of the viral protein gene closest to the 3'-end and the second closest viral gene, or between the second closest and the third closest viral protein genes to the 3'-end. For example, in typical paramyxoviruses, the viral protein gene closest to the 3'-end of the genome is the N gene, the second closest gene is the P gene, and the third closest gene is the M gene. Alternatively, when a high level of expression of the introduced gene is undesirable, the gene expression level from the viral vector can be suppressed to obtain an appropriate effect, for example, by inserting the foreign gene at a site as close as possible to the 5'-side of the minus strand genome, or by selecting an inefficient transcriptional initiation sequence.

For example, a desired S sequence of minus-strand RNA virus may be used as the S sequence to be attached when a nucleic acid encoding a foreign gene is inserted into the genome. The sequence 3'-UCCCWVWWC-5' (W= A or C; V= A, C, or G)(SEQ ID NO: 6) can preferably be used in the case of Sendai virus. Particularly preferred sequences are 3'-UCCCAGUUUC-5' (SEQ ID NO: 7), 3'-UCCCACUUAC-5' (SEQ ID NO: 8), and 3'-UCCCACUUUC-5' (SEQ ID NO: 9). When shown as DNA sequences encoding the plus strand these sequences are 5'-AGGGTCAAAG-3' (SEQ ID NO: 10), 5'-AGGGTGAATG-3' (SEQ ID NO: 11), and 5'-AGGGTGAAAG-3' (SEQ ID NO: 12). A preferred E sequence of Sendai viral vector includes, for example, 3'-AUUCUUUUU-5' (SEQ ID NO: 13) (5'-TAAGAAAAA-3' (SEQ ID NO: 14) for a DNA encoding the plus strand). An I sequence may comprise, for example, any three nucleotides, specifically 3'-GAA-5' (5'-CTT-3' in the plus strand DNA).

To prepare a virus of the present invention, a DNA encoding a genomic RNA of a minus-strand RNA virus of this invention or a complementary strand thereof is transcribed in mammalian cells, in the presence of viral proteins (i.e., N, P, and L proteins) essential for reconstitution of an RNP, which comprises a genomic RNA of a minus-strand RNA virus. Viral RNP can be reconstituted by producing either the minus strand genome (also called the negative strand, the same antisense strand as the viral genome) or the antigenome (also called the positive strand or plus strand, the complementary strand of genomic RNA). Production of the plus strand is preferable for increased efficiency of viral reconstitution. The RNA terminals preferably reflect the terminals of the 3'-leader sequence and 5'-trailer sequence as accurately as possible, as in the natural viral genome. To accurately regulate the 5'-end of the transcript, for example, the RNA polymerase may be expressed within a cell using the recognition sequence of T7 RNA polymerase as a transcription initiation site. Furthermore, the 3'-end of the transcript is cleaved accurately by the autocleavage (Hasan, M. K. et al., J. Gen. Virol. 78: 2813-2820 (1997); Kato, A. et al., EMBO J. 16: 578-587 (1997); and Yu, D. et al., Genes Cells 2: 457-466 (1997)).

For example, a recombinant Sendai virus can be constructed as follows, according to descriptions in: Hasan, M. K. et al., J. Gen. Virol. 78: 2813-2820 (1997); Kato, A. et al., EMBO J. 16: 578-587 (1997); Yu, D. et al., Genes Cells 2: 457-466 (1997); or the like.

A DNA that encodes a foreign gene partially modified to alter the portion of E sequence *(e.g.,* 5'-AGA₅₋₆C-3' site) is synthesized such that there are restriction sites for cloning at both ends. The synthetic DNA should contain an E-I-S sequence such that the E-I-S sequence is placed between the inserted foreign gene and ORFs of the flanking viral genes in the viral genome. The synthetic DNA is designed such that its size (the number of nucleotides, including the added E-I-S sequences) is a multiple of 6 when inserted into the vector (the so-called "rule of six"; Kolakofski, D. et al., J. Virol. 72:891-899 (1998); Calain, P. and Roux, L., J. Virol. 67:4822-4830 (1993); Calain, P. and Roux, L., J. Virol. 67: 4822-4830 (1993)). As the E-I-S sequence, for example, S, I, and E sequences of the minus strand of Sendai virus, for example, the sequences 5'-CTTTCACCCT-3' (SEQ ID NO: 15), 5'-AAG-3', and 5'-TTTTTCTTACTACGG-3' (SEQ ID NO: 16) respectively, are placed at the 3' end of the oligo DNA insert fragment. This fragment is inserted into a cDNA encoding the viral genome. For example, the fragment is inserted between the ORFs encoding the viral proteins in the genome and the upstream (3'-side of the genome) S sequence so that E-I-S sequences are placed one by one between the ORFs of the viral protein genes on either side of the foreign gene.

For example, a cDNA for a recombinant Sendai virus genome can be constructed by methods described in previous reports (Yu, D. et al., Genes Cells 2: 457-466 (1997); Hasan, M. K. et al., J. Gen. Virol. 78: 2813-2820 (1997)). For example, a double-stranded DNA is synthesized to have an E-I-S sequence attached to the 3'end of the sense strand of the foreign gene. The DNA is inserted immediately 3' of a desired S sequence in a cDNA encoding the plus strand of the genome. For example, a restriction site is first placed between a sequence encoding a desired viral protein gene and an S sequence involved in the transcription of the gene in a cDNA encoding the plus strand genome. A DNA encoding a foreign gene-E-I-S sequence can then be inserted into the restriction site (Tokusumi, T. et al. Virus Res 86(1-2), 33-8 (2002)).

A virus of this invention can be reconstituted by transcribing a DNA encoding a genomic RNA of a recombinant minus-strand RNA virus thus prepared, in cells in the presence of the above-described viral proteins (L, P, and N). The present invention provides DNAs encoding the viral genomic RNAs of the viruses of this invention or the complementary RNAs thereof, for manufacturing the viruses of this invention. This invention also relates to the use of DNAs encoding the genomic RNAs of the viruses, or the complementary RNAs thereof, for their application to the manufacture of the viruses of this invention. The recombinant viruses can be reconstituted by methods known in the art (WO97/16539; WO97/16538; Durbin, A. P. et al., Virology 235: 323-332 (1997); Whelan, S. P. et al., Proc. Natl. Acad. Sci. USA 92: 8388-8392 (1995); Schnell. M. J. et al., EMBO J. 13: 4195-4203 (1994); Radecke, F. et al., EMBO J. 14: 5773-5784 (1995); Lawson, N. D. et al., Proc. Natl. Acad. Sci. USA 92: 4477-4481; Garcin, D. et al., EMBO J. 14: 6087-6094 (1995); Kato, A. et al., Genes Cells 1: 569-579 (1996); Baron, M. D. and Barrett, T., J. Virol. 71:1265-1271 (1997); Bridgen, A. and Elliott, R. M., Proc. Natl. Acad. Sci. USA 93: 15400-15404 (1996)). With these methods, minus strand RNA viruses including parainfluenza virus, vesicular stomatitis virus, rabies virus, measles virus, rinderpest virus, and Sendai virus can be reconstituted from DNA. The viruses of this invention can be reconstituted according to these methods. When a viral genome-encoding DNA is made F gene, HN (or H) gene, and/or M gene deficient, such DNAs do not form infectious virions as is. However, by separately introducing host cells with these lacking genes, and/or genes encoding the envelope proteins of other viruses, and then expressing these genes therein, it is possible to form infectious virions (Hirata, T. et al., 2002, J. Virol. Methods, 104:125-133; Inoue, M. et al., 2003, J. Virol. 77:6419-6429).

Specifically, the viruses can be prepared by the steps of: (a) transcribing DNAs encoding minus-strand RNA viral genomic RNAs (minus strand RNAs), or complementary strands thereof (plus strands), in cells expressing N, P, and L proteins; and (b) harvesting complexes that comprise the genomic RNAs from these cells, or from culture supernatants thereof. For transcription, a DNA encoding a genomic RNA is linked downstream of an appropriate promoter. The genomic RNA thus transcribed is replicated in the presence of N, L, and P proteins to form an RNP complex. Then, in the presence of M, HN (or H), and F proteins, virions enclosed in an envelope are formed. For example, a DNA encoding a genomic RNA can be linked downstream of a T7 promoter, and transcribed to RNA by T7 RNA polymerase. Any desired promoter, other than those comprising a T7 polymerase recognition sequence, can be used as a promoter. Alternatively, RNA transcribed *in vitro* may be transfected into cells.

Enzymes essential for the initial transcription of genomic RNA from DNA, such as T7 RNA polymerase, can be supplied by transducing the plasmid or viral vectors that express them, or, for example, by incorporating a gene thereof into a chromosome of the cell so as to enable induction of their expression, and then inducing expression at the time of viral reconstitution. Further, genomic RNA and viral proteins essential for viral reconstitution are supplied, for example, by transducing the plasmids that express them.

Methods for transducing DNAs expressing the genomic RNAs into cells include, for example, (i) methods for making DNA precipitates which target cells can internalize; (ii) methods for making complexes comprising DNAs suitable for internalization by target cells, and comprising positive charge characteristics with low cytotoxicity; and (iii) methods for using electric pulses to instantaneously bore pores in the target cell membrane, of sufficient size for DNA molecules to pass through.

For (ii), various transfection reagents can be used. For example, DOTMA (Roche), Superfect (QIAGEN #301305), DOTAP, DOPE, DOSPER (Roche #1811169), and such can be cited. As (i), for example, transfection methods using calcium phosphate can be cited, and although DNAs transferred into cells by this method are internalized by phagosomes, a sufficient amount of DNA is known to enter the nucleus (Graham, F. L. and Van Der Eb, J., Virology 52: 456 (1973); Wigler, M. and Silverstein, S., Cell 11: 223 (1977)). Chen and Okayama investigated the optimization of transfer techniques, reporting that (1) incubation conditions for cells and coprecipitates are 2 to 4% CO₂, 35°C, and 15 to 24 hours, (2) the activity of circular DNA is higher than linear DNA, and (3) optimal precipitation is obtained when the DNA concentration in the precipitate mixture is 20 to 30 µg/ml (Chen, C. and Okayama, H., Mol. Cell. Biol. 7: 2745 (1987)). The methods of (ii) are suitable for transient transfections. Methods for performing transfection by preparing a DEAE-dextran (Sigma #D-9885 M.W. 5x 10⁵) mixture with a desired DNA concentration ratio have been known for a while. Since most complexes are decomposed in endosomes, chloroquine may also be added to enhance the effect (Calos, M. P., Proc. Natl. Acad. Sci. USA 80: 3015 (1983)). The methods of (iii) are referred to as electroporation methods, and are in more general use than methods (i) and (ii) because they are not cell selective. The efficiency of these methods is supposed to be good under optimal conditions for: the duration of pulse electric current, shape of the pulse, potency of electric field (gap between electrodes, voltage), conductivity of buffer, DNA concentration, and cell density.

Of the above three categories, the methods of (ii) are simple to operate and can examine many samples using a large amount of cells, and thus transfection reagents are suitable for the transduction into cells of DNA for viral reconstitution. Preferably, Superfect Transfection Reagent (QIAGEN, Cat No. 301305), or DOSPER Liposomal Transfection Reagent (Roche, Cat No. 1811169) is used, but transfection reagents are not limited to these.

Specifically, virus reconstitution from cDNA can be carried out, for example, as follows:

In a plastic plate of about 24- to 6-wells, or a 100-mm Petri dish or the like, LLC-MK2 cells (ATCC CCL-7) derived from monkey kidney are cultured till near 100% confluent, using minium essential medium (MEM) comprising 10% fetal calf serum (FCS) and antibiotics (100 units/ml penicillin G and 100 µg/ml streptomycin), and infected with, for example, 2 plaque forming units (PFU)/ceH of the recombinant vaccinia virus vTF7-3, which expresses T7 RNA polymerase and has been inactivated by 20-minutes of UV irradiation (PLWUV) in the presence of 1 µg/ml psoralen (Fuerst, T. R. et al., Proc. Natl. Acad. Sci. USA 83: 8122-8126 (1986); Kato, A. et al., Genes Cells 1: 569-579 (1996)). The amount of psoralen added and the UV irradiation time can be appropriately adjusted. One hour after infection, 2 to 60 µg, and more preferably 3 to 20 µg, of DNA encoding the genomic RNA of a recombinant Sendai virus is transfected along with the plasmids expressing trans-acting viral proteins essential for viral RNP production (0.5 to 24 µg of pGEM-N, 0.25 to 12 µg of pGEM-P, and 0.5 to 24 µg of pGEM-L) (Kato, A. et al., Genes Cells 1: 569-579 (1996)), using the lipofection method or such with Superfect (QIAGEN). The ratio of the amounts of expression vectors encoding the N, P, and L proteins is, for example, 2:1:2; and the plasmid amounts are appropriately adjusted, for example, in the range of 1 to 4 µg ofpGEM-N, 0.5 to 2 µg of pGEM-P, and 1 to 4 µg of pGEM-L.

The transfected cells are cultured, as occasion may demand, in serum-free MEM comprising 100 µg/ml of rifampicin (Sigma) and cytosine arabinoside (AraC), more preferably only 40 µg/ml of cytosine arabinoside (AraC) (Sigma). Optimal drug concentrations are set so as to minimize cytotoxicity due to the vaccinia virus, and to maximize virus recovery rate (Kato, A. et al., Genes Cells 1: 569-579 (1996)). After culturing for about 48 to 72 hours after transfection, cells are harvested, and then disintegrated by repeating freeze-thawing three times. The disintegrated materials comprising RNP are re-infected to LLC-MK2 cells, and the cells are cultured. Alternatively, the culture supernatant is recovered, added to a culture solution of LLC-MK2 cells to infect them, and then cultured. Transfection can be conducted by, for example, forming a complex with lipofectamine, polycationic liposome, or the like, and transducing the complex into cells. Specifically, various transfection reagents can be used. For example, DOTMA (Roche), Superfect (QIAGEN #301305), DOTAP, DOPE, and DOSPER (Roche #1811169) may be cited. In order to prevent decomposition in the endosome, chloroquine may also be added (Calos, M. P., Proc. Natl. Acad. Sci. USA 80: 3015 (1983)). In cells transduced with RNP, viral gene expression from RNP and RNP replication progress, and the virus is amplified. By diluting the viral solution (the culture supernatant) thus obtained (*e.g*., 10⁶-fold), and then repeating the amplification, the vaccinia virus vTF7-3 can be completely eliminated. Amplification is repeated, for example, three or more times. Viruses thus obtained can be stored at -80°C. In order to reconstitute a virus without transmission ability, which is defective in a gene encoding an envelope protein, LLC-MK2 cells expressing the envelope protein may be used for transfection, or a plasmid expressing the envelope protein may be cotransfected. Alternatively, an envelope gene defective type virus can be amplified by culturing the transfected cells overlaid with LLK-MK2 cells expressing the envelope protein (see WO00/70055 and WO00/70070).

Titers of viruses thus recovered can be determined, for example, by measuring CIU (Cell Infectious Unit) or hemagglutination activity (HA) (WO00/70070; Kato, A. et al., Genes Cells 1: 569-579 (1996); Yonemitsu, Y. & Kaneda, Y., Hemaggulutinating virus of Japan-liposome-mediated gene delivery to vascular cells. Ed. by Baker AH. Molecular Biology of Vascular Diseases. Method in Molecular Medicine: Humana Press: pp. 295-306 (1999)). Titers of viruses carrying GFP (green fluorescent protein) marker genes and the like can be quantified by directly counting infected cells, using the marker as an indicator (for example, as GFP-CIU). Titers thus measured can be handled in the same way as CIU (WO00/70070). In some cases, viral titers may be indicated in TU (transduction unit).

As long as a virus can be reconstituted, the host cells used in the reconstitution are not particularly limited. For example, in the reconstitution of Sendai virus and such, cultured cells such as LLC-MK2 cells, CV-1 cells (ATCC CCL-70) derived from monkey kidney, and BHK cells (*e.g*., ATCC CCL-10) derived from hamster kidney, and cells derived from humans can be used. By expressing suitable envelope proteins in these cells, infectious virions having the proteins in envelopes can also be obtained. Further, to obtain a large quantity of a Sendai virus, a virus obtained from an above-described host can be infected to embryonated hen eggs, to amplify the virus. Methods for manufacturing viruses using hen eggs have already been developed (Nakanishi, et al., ed., "State-of-the-Art Technology Protocol in Neuroscience Research III, Molecular Neuron Physiology", Koseisha, Osaka, pp. 153-172 (1993)). Specifically, for example, a fertilized egg is placed in an incubator, and cultured for nine to twelve days at 37 to 38°C to grow an embryo. After the virus is inoculated into the allantoic cavity, the egg is cultured for several days (for example, three days) to proliferate the virus. Conditions such as the period of culture may vary depending upon the recombinant Sendai virus being used. Then, allantoic fluids comprising the vector are recovered. Separation and purification of a Sendai virus from allantoic fluids can be performed according to a usual method (Tashiro, M., "Virus Experiment Protocol", Nagai, Ishihama, ed., Medical View Co., Ltd., pp. 68-73 (1995)).

For example, the construction and preparation of Sendai virus defective in F gene can be performed as described below (see WO00/70055 and WO00/70070):

### <1> Construction of a genomic cDNA of an F defective Sendai virus, and a plasmid expressing F gene

A full-length genomic cDNA of Sendai virus (SeV), the cDNA of pSeV18⁺ b(+) (Hasan, M. K. et al., J. General Virology 78: 2813-2820 (1997)) ("pSeV18⁺ b(+)" is also referred to as "pSeV18⁺"), is digested with *Sph*I*lKpn*I to recover a fragment (14673 bp), which is cloned into pUC18 to prepare plasmid pUC18/KS. Construction of an F gene defective site is performed on this pUC18/KS. An F gene deficiency is created by a combination of PCR-ligation methods, and, as a result, the F gene ORF (ATG-TGA = 1698 bp) is removed. Then, for example, atgcatgccggcagatga (SEQ ID NO: 17) is ligated to construct an F gene defective type SeV genomic cDNA (pSeV18⁺/ΔF). A PCR product formed in PCR by using the pair of primers [forward: 5'-gttgagtactgcaagagc/SEQ ID NO: 18, reverse: 5'-tttgccggcatgcatgtttcccaaggggagagttttgcaacc/SEQ ID NO: 19] is connected upstream of F gene, and a PCR product formed using the pair of primers [forward: 5'-atgcatgccggcagatga/SEQ ID NO: 20, reverse: 5'-tgggtgaatgagagaatcagc/SEQ ID NO: 21] is connected downstream of F gene at EcoT22I. The plasmid thus obtained is digested with *Sac*I and *Sal*I to recover a 4931 bp fragment of the region comprising the F defective site, which is cloned into pUC 18 to form pUClB/dFSS. This pUC18/dFSS is digested with *Dra*III, the fragment is recovered, replaced with the *Dra*III fragment of the region comprising the F gene of pSeV18⁺, and ligated to obtain the plasmid pSeV 18⁺/ΔF. A foreign gene is inserted, for example, in to the *Nsi*I and *Ngo*MIV restriction enzyme sites in the F gene defective site of pUC18/dFSS.

### <2> Preparation of helper cells that induce SeV-F protein expression

To construct an expression plasmid of the Cre/loxP induction type that expresses the Sendai virus F gene (SeV-F), the SeV-F gene is amplified by PCR, and inserted to the unique *Swa*I site of the plasmid pCALNdlw (Arai, T. et al., J. Virology 72, p1115-1121 (1998)), which is designed to enable the inducible expression of a gene product by Cre DNA recombinase, thus constructing the plasmid pCALNdLw/F.

To recover infectious virions from the F gene defective genome, a helper cell line expressing SeV-F protein is established. The monkey kidney-derived LLC-MK2 cell line, which is commonly used for SeV proliferation, can be used as the cells, for example. LLC-MK2 cells are cultured in MEM supplemented with 10% heat-treated inactivated fetal bovine serum (FBS), penicillin G sodium (50 units/ml), and streptomycin (50 µg/ml) at 37°C in 5% CO₂. Since the SeV-F gene product is cytotoxic, the above-described plasmid pCALNdLw/F, which was designed to enable inducible expression of the F gene product with Cre DNA recombinase, is transfected to LLC-MK2 cells for gene transduction by the calcium phosphate method (using a mammalian transfection kit (Stratagene)), according to protocols well know in the art.

The plasmid pCALNdLw/F (10 µg) is transduced into LLC-MK2 cells grown to 40% confluency using a 10-cm plate, and the cells are then cultured in MEM (10 ml) comprising 10% FBS, in a 5% CO₂ incubator at 37°C for 24 hours. After 24 hours, the cells are detached and suspended in the medium (10 ml). The suspension is then seeded onto five 10-cm dishes, 5 ml to one dish, 2 ml each to two dishes, and 0.2 ml each to two dishes, and cultured in MEM (10 ml) comprising G418 (GIBCO-BRL) (1200 µg/ml) and 10% FBS. The cells were cultured for 14 days, exchanging the medium every two days, to select cell lines stably transduced with the gene. The cells grown from the above medium that show the G418 resistance are recovered using a cloning ring. Culture of each clone thus recovered is continued in 10-cm plates until confluent.

After the cells have grown to confluency in a 6-cm dish, F protein expression is induced by infecting the cells with Adenovirus AxCANCre, for example, at MOI = 3, according to the method of Saito, *et al.* (Saito et al., Nucl. Acids Res. 23: 3816-3821 (1995); Arai, T. et al., J. Virol 72, 1115-1121 (1998)).

### <3> Reconstitution and amplification of F gene defective Sendai virus (SeV)

The above-described plasmid pSeV18⁺/ΔF, into which a foreign gene has been inserted, is transfected to LLC-MK2 cells as follows: LLC-MK2 cells are seeded at 5x 10⁶ cells/dish in 100-mm dishes. When genomic RNA transcription is carried out with T7-RNA polymerase, cells are cultured for 24 hours, then infected at an MOI of about two for one hour at room temperature, with recombinant vaccinia virus expressing T7-RNA polymerase, which has been treated with psoralen and long-wave ultraviolet rays (365 nm) for 20 minutes (PLWUV-VacT7: Fuerst, T. R. et al., Proc. Natl. Acad. Sci. USA 83, 8122-8126 (1986)). For the ultraviolet ray irradiation of vaccinia virus, for example, an UV Stratalinker 2400 equipped with five 15-watt bulbs can be used (catalogue No. 400676 (100V), Stratagene, La Jolla, CA, USA). The cells are washed with serum-free MEM, then an appropriate lipofection reagent is used to transfect the cells with a plasmid expressing the genomic RNA, and expression plasmids expressing the N, P, L, F, and HN proteins of SeV. The ratio of amounts of these plasmids can be preferably set as 6:2:1:2:2:2, in this order, though is not limited thereto. For example, a genomic RNA-expressing plasmid as well as expression plasmids expressing the N, P, L, and F plus HN proteins (pGEM/NP, pGEM/P, pGEM/L, and pGEM/F-HN; WO00/70070, Kato, A. et al., Genes Cells 1, 569-579 (1996)) are transfected at an amount ratio of 12 µg, 4 µg, 2 µg, 4 µg, and 4 µg/dish, respectively. After culturing for several hours, the cells are twice washed with serum-free MEM, and cultured in MEM comprising 40 µg/ml of cytosine β-D-arabinofuranoside (AraC: Sigma, St. Louis, MO) and 7.5 µg/ml of trypsin (Gibco-BRL, Rockville, MD). These cells are recovered, and the pellets are suspended in Opti-MEM (10⁷ cells/ml). Suspensions are freeze-thawed three times, mixed with lipofection reagent DOSPER (Boehringer Mannheim) (10⁶ cells/25 µl DOSPER), stood at room temperature for 15 minutes, transfected to the above-described cloned F-expressing helper cells (10⁶ cells/well in a 12-well-plate), and cultured in serum-free MEM (comprising 40 µg/ml AraC and 7.5 µg/ml trypsin) to recover the supernatant. Viruses defective in a gene other than F, for example, the HN or M gene, can also be prepared by similar methods to this.

When a viral gene-defective type virus is prepared, for example, if two or more different kinds of viruses, that comprise the different viral genes which are defective in the viral genome in the viruses, are transduced into the same cell, the viral proteins that are defective in each of the viruses are supplied by their expression from the other vectors. Thus, together, these viruses make up for protein deficiencies, and infectious virions can be formed. As a result, the replication cycle can amplify the viruses. In other words, when two or more kinds of viruses of this invention are inoculated in a combination that together supplements deficient viral proteins, mixtures of viruses defective in each of the viral genes can be produced on a large scale and at a low cost. When compared to viruses that are not deficient in viral genes, these viruses have smaller genomes, due to deficient viral genes, and can thus carry larger foreign genes. Further, these viruses, which lack proliferative ability due to deficient viral genes, are extracellularly attenuated, and maintaining coinfection is difficult. They are therefore sterilized, which is an advantage in environmental release management.

When, after administering a transmissible minus-strand RNA viral vector to an individual or cell, the proliferation of the viral vector must be restrained due to treatment completion and such, it is also possible to specifically restrain only the proliferation of the viral vector, with no damage to the host, by administering an RNA-dependent RNA polymerase inhibitor.

The foreign genes to be carried by viral vectors of the present invention are not particularly limited, and may encode natural proteins including hormones, cytokines, growth factors, receptors, intracellular signaling molecules, enzymes, and peptides. Such proteins include secretory proteins, membrane proteins, cytoplasmic proteins, and nuclear proteins. The foreign genes may also artificial proteins including fusion proteins, such as chimeric toxins; dominant-negative proteins (including soluble receptor molecules and dominant-negative membrane-bound receptors); truncated cell-adhesion molecules; cell surface molecules; and antibody fragments. Proteins to which a secretory signal, membrane-targeting signal, or nuclear localization signal has been added are also included. The function of a particular gene can be suppressed by introducing and expressing a foreign gene such as an antisense RNA, molecule or RNA-cleaving ribozyme, siRNA (small interfering RNAs), or such. The foreign gene may also be a marker gene used to evaluate the efficiency of gene introduction, expression stability, or such.

According to the methods also disclosed herein, the viruses of this invention can be released into the culture medium of virus-producing cells, for example, at a titer of 1x 10⁵ CIU/ml or more, preferably 1x 10⁶ CIU/ml or more, more preferably 5x 10⁶ CIU/ml or more, more preferably 1x10⁷ CIU/ml or more, more preferably 5x 10⁷ CIU/ml or more, more preferably 1x10⁸ CIU/ml or more, and more preferably 5x 10⁸ CIU/ml or more. Viral titers can be measured by a method described in this description and others (Kiyotani, K. et al., Virology 177(1), 65-74 (1990); WO00/70070).

The recovered minus-strand RNA viruses can be purified to become substantially pure. Purification can be performed by purification and separation methods known in the art, including filtration, centrifugal separation, and column purification, or any combinations thereof. "Substantially pure" means that a virus accounts for a major proportion of a sample in which the virus exists as a component. Typically, a substantially pure virus can be identified by confirming that the proportion of proteins derived from the virus is, for example, 10% or more of all of the proteins in a sample, preferably 20% or more, preferably 50% or more, preferably 70% or more, more preferably 80% or more, and further more preferably 90% or more (excluding, however, proteins and such added as carriers, stabilizers, etc.). Examples of specific methods for purifying paramyxoviruses are those that use cellulose sulfate ester or cross-linked polysaccharide sulfate ester (Japanese Patent Publication Nos. JP62030752B, JP62033879B, and JP62030753B), and those for methods for adsorbing them to polysaccharides comprising fucose sulfate and/or degradation products thereof (WO97/32010).

The present invention provides a pharmaceutical composition comprising the virus provided by the present invention. In preparing compositions comprising the virus of the present invention, the virus can be combined with a pharmaceutically acceptable desired carrier or vehicle, as necessary. "A pharmaceutically acceptable carrier or vehicle" means a material that can be administered together with the virus which does not significantly inhibit gene transduction via the virus. For example, a virus can be appropriately diluted with physiological saline or phosphate-buffered saline (PBS) to form a composition. When a virus is grown in hen eggs or the like, the "pharmaceutically acceptable carrier or vehicle" may comprise allantoic fluids. Further, compositions comprising a virus may include carriers or vehicles such as deionized water and 5% dextrose aqueous solution. Furthermore, compositions may comprise, besides the above, plant oils, suspending agents, surfactants, stabilizer, biocides, and so on. The compositions can also comprise preservatives or other additives. The compositions comprising the viruses of this invention are useful as reagents and medicines. The present invention provides the use of the virus provided by the present invention for the preparation of a pharmaceutical composition for gene therapy.

The viruses of the invention can be used as gene therapy vectors for use in in vivo and ex vivo administration to the body. When the minus-strand RNA virus of the present invention is administered to the living body, the dose may vary depending upon the disorder, body weight, age, gender, and-symptoms of patients, as well as purpose of administration, form of the composition to be administered, administration method, gene to be transduced, and so on; however, those skilled in the art can appropriately determine dosages. Administration route can be appropriately selected, although administration can be performed, for example, percutaneously, intranasally, perbronchially, intramuscularly, intraperitoneally, intravenously, intra-articularly, intraspinally, or subcutaneously, but is not limited to these routes. Administration can also be performed locally or systemically. Doses of the virus are preferably administered in a pharmaceutically acceptable carrier in a range of preferably about 10⁵ CIU/ml to about 10¹¹ CIU/ml, more preferably about 10⁷ CIU/ml to about 10⁹ CIU/ml, most preferably about 1x 10⁸ CIU/ml to about 5x 10⁸ CIU/ml. In humans, a single dose is preferably in the range of 2x 10⁵ CIU to 2x 10¹⁰ CIU, and can be administered once or more, so long as the side effects are within a clinically acceptable range. The same applies to the number of administrations per day. With animals other than humans, for example, the above-described doses can be converted based on the body weight ratio or volume ratio of a target site for administration (*e.g*. average values) between the objective animal and humans, and the converted doses can be administered to the animals. Subjects for administering compositions comprising the viruses of this invention include all mammals, such as humans, monkeys, mice, rats, rabbits, sheep, cattle, and dogs.

### Brief Description of the Drawings

Fig. 1 is a diagram showing the two hypermutable regions of the hCFTR gene. The sequences are numbered with nucleotide A of the start codon ATG in hCFTR as position +1. Of the sequences in this diagram, the normal and mutant sequences of the position 1257 region are shown as SEQ ID NOs: 50 and 51, respectively; and the normal and mutant sequences of the position 3905 region are shown as SEQ ID NOs: 52 and 53, respectively.
Fig. 2 is a diagram showing a sequence comparison between the two hypermutable regions of the hCFTR gene and the EI sequence of Sendai virus cDNA. Of the sequences in this diagram, NP and L are shown in SEQ ID NO: 54; P and HN are shown in SEQ ID NO: 55; M and F are shown in SEQ ID NO: 56; and the position 1257 and position 3905 regions are shown in SEQ ID NOs: 50 and 52, respectively.

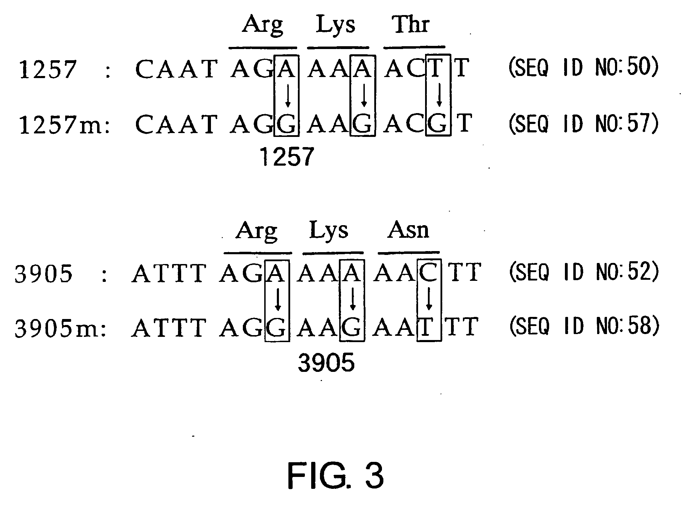

Fig. 3 is a diagram showing alterations in the hypermutable regions of the hCFTR gene. Of the sequences in this diagram, "1257" and "1257m" are shown as SEQ ID NOs: 50 and 57, respectively; and "3905" and "3905m" are shown as SEQ ID NOs: 52 and 58, respectively.

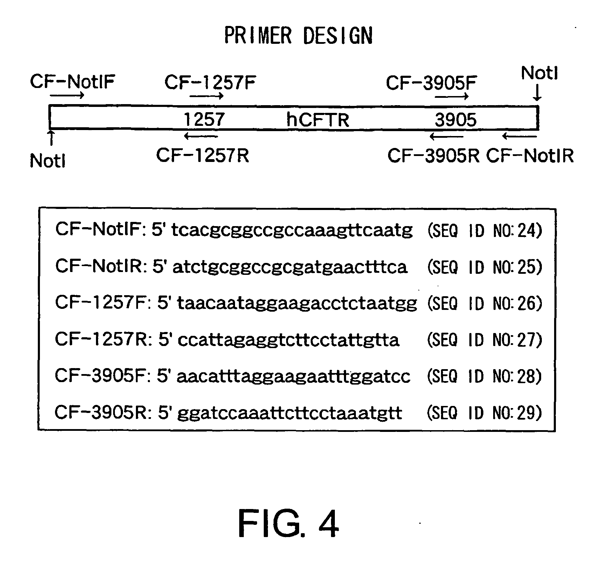

Fig. 4 is a diagram showing the primers designed for use in altering the hypermutable regions of the hCFTR gene. The sequences are shown from the top as SEQ ID NOs: 24 to 29 in this diagram.

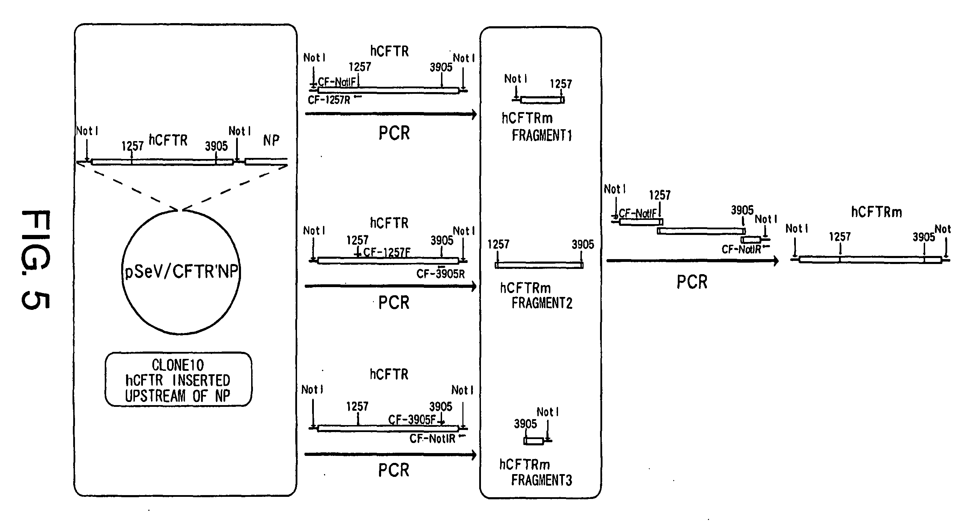

Fig. 5 is a schematic diagram showing the construction of a gene fragment (hCFTRm) of the hCFTR gene whose hypermutable regions have been altered.

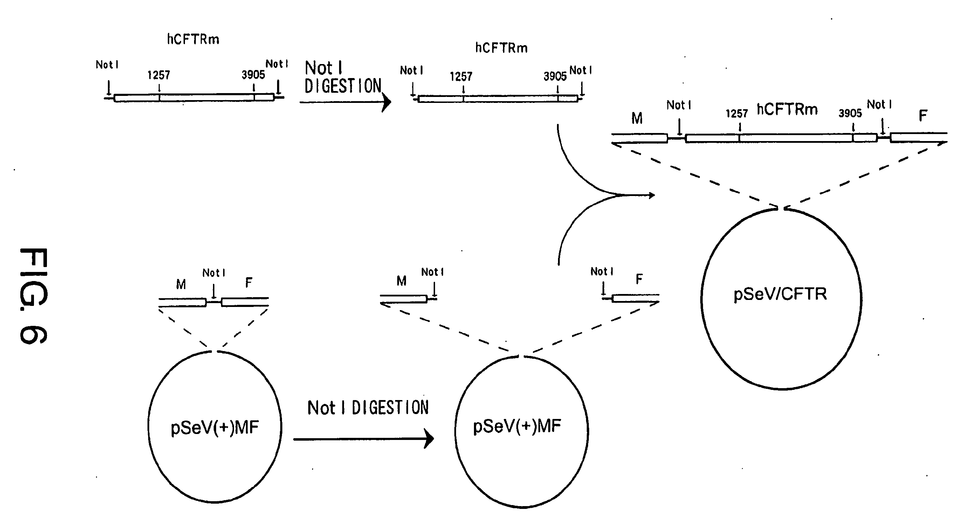

Fig. 6 is a schematic diagram showing the construction of a cDNA (pSeV/CFTR) encoding a SeV genome, where hCFTRm has been inserted between the M gene and the F gene.

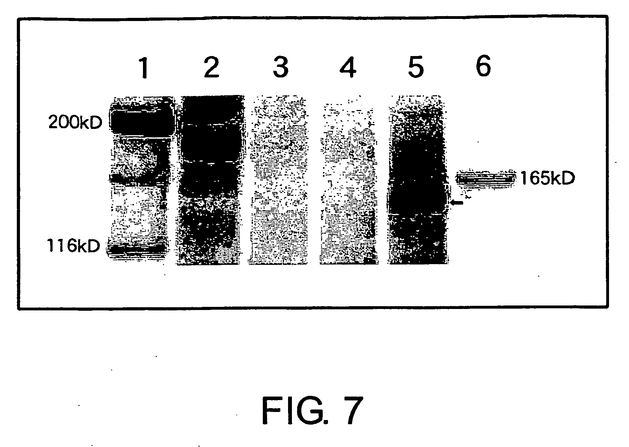

Fig. 7 is a photograph demonstrating the expression of hCFTR protein. The photograph shows the result of Western blotting using a monoclonal anti-human CFTR (C terminus-specific) antibody (Genzyme) as a primary antibody. Lane 1: molecular-weight marker; lane 2: T84 cells (positive control); lane 3: BHK-21 cells (negative control 1); lane 4: wild-type SeV-infected BHK-21 cells (negative control 2); lane 5: SeV/CFTR-infected BHK-21 cells; lane 6: molecular-weight marker.

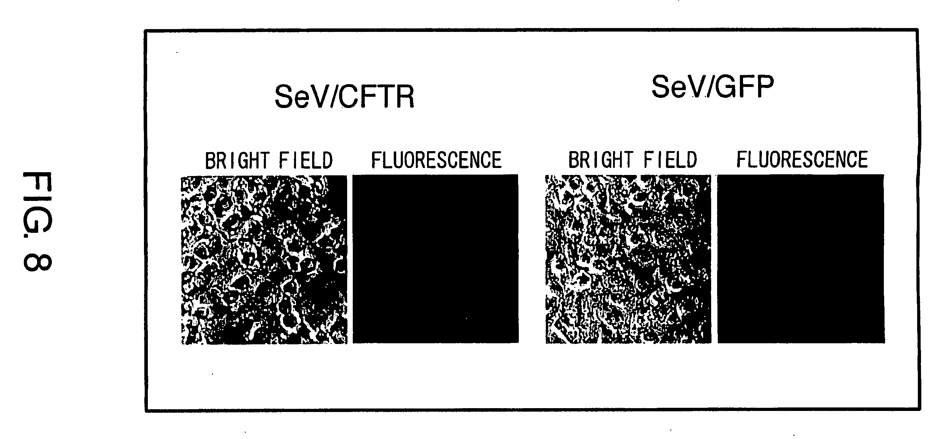

Fig. 8 is a photograph demonstrating the expression of hCFTR protein. The photograph shows the result of immunocytochemistry analysis. A mouse anti-human CFTR monoclonal antibody (C terminus-specific; 10 µg/ml; CHEMICON) was used as the primary antibody, and an Alexa Fluor 594-conjugated goat anti-mouse IgG (H+L) (5 µg/ml; Molecular Probes) was used as the secondary antibody. Transmissable SeV carrying a GFP gene (SeV/GFP) was used as a control.

### Best Mode for Carrying out the Invention

Hereinafter, the present invention will be explained in more detail with reference to Examples, but is not to be construed as being limited thereto.

[Example 1] SeV vector carrying an hCFTR gene whose hypermutable regions have been altered In the instant example, SeV vector carrying an hCFTR gene with altered hypermutable regions was constructed using human CFTR (hCFTR) as a hypermutable region-comprising gene. When the full-length sequence of the normal (wild-type) hCFTR gene is carried by a Sendai virus vector, nucleotide mutations are generated with high frequency in two regions that coincide with 5'-AGA₅₋₆C-3', and these nucleotide mutations change the amino acid sequence and result in the loss of hCFTR protein activity (Fig. 1). The nucleotide sequences of these two regions in the hCFTR gene were then artificially altered to reduce their homology to the EI sequence without altering the hCFTR amino acid sequence. The altered gene was inserted into a Sendai virus vector. There were no mutations observed in the hCFTR gene of the genome when this Sendai virus vector which carries the human CFTR gene with altered hypermutable regions was used. In addition, normal hCFTR protein activity was detected both *in vitro* and *in vivo.*

### 1. Alteration of the hypermutable regions of hCFTR gene

(1) PCR was carried out using a pQB6.2 plasmid comprising an ORF of the hCFTR gene as a template and *Not*I-tailed primers: CFTR-N (5'-acttgcggccgccaaagttcaatgcagaggtcgcctctggaaaaggccagc/SEQ ID NO: 22) and CFTR-C (5'-atccgcggccgcgatgaactttcaccctaagtttttcttactacggctaaagccttgtatcttgcacctcttcttc/SEQ ID NO: 23) for the 5' and 3' ends, respectively. The resulting PCR products were digested with the restriction enzyme *Not*I and recovered, and then inserted into the *Not*I site of the full-length SeV genome cDNA pSeV18+ (Kato, A. et al., Genes Cells 1, 569-579 (1996)) to obtain pSeV/CFTR'NP.
(2) Primers were designed in such a way that the amino acid sequence of hCFTR in the hypermutable regions remains unchanged (Figs. 3 and 4).
   CF-NotIF: 5' tcacgcggccgccaaagttcaatg (SEQ ID NO: 24)
   CF-NotIR: 5' atctgcggccgcgatgaactttca (SEQ ID NO: 25)
   CF-1257F: 5' taacaataggaagacctctaatgg (SEQ ID NO: 26)
   CF-1257R: 5' ccattagaggtcttcctattgtta (SEQ ID NO: 27)
   CF-3905F: 5' aacatttaggaagaatttggatcc (SEQ ID NO: 28)
   CF-3905R: 5' ggatccaaattcttcctaaatgtt (SEQ ID NO: 29)
(3) Using pSeV/CFTR'NP as a template, the hCFTRm fragments 1, 2, and 3 were amplified with CF-NotIF and CF-1257R, CF-1257F and CF-3905R, and CF-3905F and CF-NotIR, respectively (Fig. 5). The enzyme used was KOD plus. PCR was carried out using 50 µl of PCR reaction solution comprising 5 µl of 10x buffer, 5 µl of dNTP, 2 µl of MgSO₄ (final concentration was 1 mM), primers (30 µmol each), 1 µl of KOD plus, and 5 µl of template (50 ng). The cycle conditions were: 98°C for 2 min; 35 cycles of 98°C for 15 sec, 55°C for 30 sec, and 68°C for 3 min; and 72°C for 7 min, followed by 4°C storage.
(4) The hCFTRm/NotI fragment was amplified by PCR using hCFTRm fragments 1, 2, and 3 as templates and the CF-NotIF and CF-NotIR primers (Fig. 5). The enzyme used was KOD plus. PCR was carried out using 50 µl of PCR solution comprising 5 µl of 10x buffer, 5 µl of dNTP, 3 µl of MgSO₄ (final concentration was 1.5 mM), primers (30 µmol each), 1 µl of KOD plus, and 6 µl of template (50 ng). The cycle conditions were: 98°C for 3 min; 35 cycles of 98°C for 15 sec, 55°C for 30 sec, and 68°C for 5 min; and 72°C for 7 min, followed by storage at 4°C.

### 2. Construction of a SeV cDNA plasmid carrying hCFTR with altered hypermutable regions

After the *Not*I treatment, the hCFTRm/NotI fragment was inserted into the *Not*I site between the M and F genes of a SeV cDNA (pSeV(+)MF; Tokusumi, T. et al., 2002, Virus. Res. 86:33-38) by ligation to construct a SeV/CFTR cDNA plasmid (pSeV/CFTR) (Fig. 6).

### 3. Reconstitution of SeV carrying the altered full-length hCFTR gene

Viral reconstitution was performed using the SeV cDNA carrying the altered full-length hCFTR gene (named hCFTRm), and the resulting viral vector was recovered. The reconstitution protocol is described below.

BHK-21 cells cultured in 6-well plates were washed with serum-free MEM (GIBCO/11668-019). 200 µl of Opti-MEM containing 0.85% FBS (v/v) and 3x 10⁶ pfu of a T7 RNA polymerase-expressing recombinant vaccinia virus (PLWUV-vTF7-3) that has been treated with long wavelength ultraviolet light (PLWUV) were added to each well, and then the plates were incubated for 1 hour to establish infection. 5 µg of cDNA, 1 µg of pGEM/NP, 0.5 µg of pGEM/P, and 1 µg of pGEM/L were suspended in 250 µl Opti-MEM (supplement-free stock solution). 15 µl of the LipofectAMINE 2000 reagent [GIBCO/11668-019] was suspended in 250 µl Opti-MEM (supplement-free stock solution). The two solutions were then mixed, and the resulting mixture was allowed to stand at room temperature for 20 to 30 minutes. A transfection solution was prepared by the procedure described above. After 1 hour of infection with PLWUV-vTF7-3, the cells were washed twice with Opti-MEM (supplement-free stock solution). Then, 0.5 ml/well of Opti-MEM containing 10% FBS was mixed with the transfection solution, and 1 ml of this mixture was added to the cells in each well. After 6 to 8 hours of incubation at 37°C, Glasgow MEM (containing 10% FBS and 80 µg/ml AraC) was added and cultured overnight at 37°C.

The medium was removed the next day, and then the cells were washed once with serum-free MEM. 1 ml of serum-free medium VP-SFM (containing 7.5 µg/ml trypsin and 40 µg/ml AraC) was added to the cells in each well. After 6 hours, the cells in each well were overlaid with 1 ml of Glasgow MEM (5x 10⁵ LLC-MK₂ cells/ml, 10% FBS, and 40 µg/ml AraC).

Two days later, the cells were harvested from three wells and centrifuged at 3000 rpm for 1 min. Then, excess medium was aspirated until 0.45 ml of the medium remained. The precipitated cells were suspended by vortexing and frozen in an ethanol-dry ice bath. After 5 minutes, the cells were thawed at 37°C and then vortexed. This freeze-thawing cycle was repeated three times in total. The cell suspensions which had been treated with freeze-thawing were inoculated into three embryonated hen eggs (150-µl/egg) that had been cultured for ten days.

The inoculated hen eggs were cultured for another three days. Then, the eggs were cooled at 4°C for three hours, from which chorioallantoic fluid was collected. The presence of virus was confirmed by HA assay. The collected chorioallantoic fluid was inoculated into three embryonated hen eggs (150-µl/egg) that had been cultured for ten days. These treatments were repeated once or twice until a positive result was obtained in the HA assay. As a result, 2⁶ viruses or more were detected by the HA assay. The recombinant virus is named SeV/CFTR.

### 4. Confirmation of the full-length hCFTR gene sequence in viral genome

RNA genome was extracted from the virus, and a fragment comprising hCFTR was amplified by RT-PCR to confirm the full-length hCFTR sequence. RNA genome extraction and reverse transcription were carried out according to the QIAamp Viral RNA Mini Spin Protocol and the reverse transcription protocol for ReverTra Ace-α-^{™} (TOYOBO), respectively. PCR was carried out using the RT products as the template and the following primer sets:
Primer set 1: SeVF4003/hCFTR-C4
   SeVF4003: 5'-cggtgaggaggactgttcgagc (SEQ ID NO: 30)
   hCFTR-C4: 5'-cagttcagtcaagtttgcct (SEQ ID NO: 31)
Primer set 2: hCFTR-N4/SeVR4993
   hCFTR-N4: 5'-cgaccaatttagtgcagaa (SEQ ID NO: 32)

   SeVR4993: 5'-ttcccttcatcgactatgacc (SEQ ID NO: 33)
Primers for the full-length hCFTR sequence:
   Forward:
      SeVF4003: 5'-cggtgaggaggactgttcgagc (SEQ ID NO: 30)
      hCFTR-N0: 5'-atgcagaggtcgcctctggaaaag (SEQ ID NO: 34)
      hCFTR-N1: 5'-cacattggaatgcagatgaga (SEQ ID NO: 35)
      hCFTR-N2: 5'-tatctgtgcttccctatgca (SEQ ID NO: 36)
      hCFTR-N3: 5'-gcacagtggaagaatttca (SEQ ID NO: 37)
      hCFTR-N4: 5'-cgaccaatttagtgcagaa (SEQ ID NO: 32)
      hCFTR-N5: 5'-ggagtgcctttttgatgat (SEQ ID NO: 38)
      hCFTR-N6: 5'-ggatgaccttctgcctctta (SEQ ID NO: 39)
      hCFTR-N7: 5'-ggatagcttgatgcgatctgt (SEQ ID NO: 40)
      hCFTR-N8: 5'-ggaaagttgcagatgaggtt (SEQ ID NO: 41)
   Reverse:
      SeVR4993: 5'-ttcccttcatcgactatgacc (SEQ ID NO: 33)
      hCFTR-C0: 5'-ctaaagccttgtatcttgcacctc (SEQ ID NO: 42)
      hCFTR-C1Y: 5'-acctcatctgcaactttcca (SEQ ID NO: 43)
      hCFTR-C2: 5'-catggctaaagtcaggata (SEQ ID NO: 44)
      hCFTR-C3Y: 5'-tctattaagaatcccacct (SEQ ID NO: 45)
      hCFTR-C4: 5'-cagttcagtcaagtttgcct (SEQ ID NO: 31)
      hCFTR-C5: 5'-gtctggctgtagattttgga (SEQ ID NO: 46)
      hCFTR-C6: 5'-tgaagtcttgcctgctccagt (SEQ ID NO: 47)
      hCFTR-C7Y: 5'-agtatctcacataggctgccttcc (SEQ ID NO: 48)
      hCFTR-C8: 5'-ggagcagtgtcctcacaata (SEQ ID NO: 49)
As a result, the fragment expected for each PCR was detected.

### 5. Confirmation of the in vitro expression of hCFTR protein

### (1) Western blotting (Fig. 7)

The expression of hCFTR protein from SeV carrying the altered hCFTR gene was confirmed by Western blotting. As a first step to prepare samples, SeV/CFTR was used to infect confluent BHK-21 cells (in 100-mm dishes at an MOI of 3). The hCFTR-expressing cell line T84, uninfected BHK-21 cells, BHK-21 cells infected with wild-type SeV (SeV/w; at an MOI of 3), and the like were used as controls. After 48 hours of infection with the viral vector, the cells were washed three times with ice-cold PBS(-), and lysed with lysis buffer (5 ml/dish) [10 mM Tris-HCl (pH 7.5), 2 mM EDTA, 1 mM benzamidine, 10 µg/ml leupeptin hydrochloride, 10 µg/ml aprotonin, and 2 mM PMSF (prepared at the time of use)]. After 30 seconds of centrifugation at 3500 rpm, 4°C, the supernatant was removed and the precipitated cells were suspended in 1 ml of lysis buffer. The suspension was allowed to stand at 4°C for 1 hour. After 30 minutes of centrifugation at 15000 rpm, 4°C, the supernatant was removed and the precipitate was suspended in 80 µl of solubilization buffer [lysis buffer containing 0.2% SDS (w/v) and 0.2% Triton X-100 (v/v)]. The suspension was allowed to stand at room temperature for three hours. After 30 minutes of centrifugation at 15000 rpm, 4°C, the supernatant was transferred to a new tube. After an equal volume of disaggregation buffer [50 mM Tris (pH 6.8), H₃PO₄, 2% SDS (w/v), 15% glycerol (w/v), 2% β-mercaptoethanol (v/v), 1 mM EDTA, and 0.02% (w/v) bromophenol blue] was added and mixed well with the supernatant, the resulting mixture was allowed to stand at 4°C overnight.

The sample was fractionated by SDS-PAGE and transferred onto a membrane. Then, the membrane was reacted with a C terminus-specific anti-human CFTR antibody (200x dilution; Genzyme) as the primary antibody and an HRP-conjugated anti-mouse IgG antibody [2000x dilution; Daiichi Pure Chemicals] or an HRP-conjugated anti-biotin antibody [1000x dilution; Daiichi Pure Chemicals] as the secondary antibody. The detection was carried out using ECL Detection Kit (Amersham Pharmacia Biotech).

As shown in Fig. 7, a 160-180 kDa hCFTR protein band was detected in T84 cells used as a positive control for hCFTR protein expression, while a 140 kDa hCFTR protein was detected in BHK-21 cells infected with SeV carrying the altered hCFTR gene. A similar result was also obtained when cells such as LLC-MK₂ and BEAS-2B were used.

### (2) Immunocytochemistry (Fig. 8)

The expression of hCFTR protein from SeV carrying the altered hCFTR gene was confirmed by the following method using immunostaining. Confluent LLC-MK₂ cells were infected with SeV/CFTR (at an MOI of 3). LLC-MK₂ cells infected with SeV/GFP (at an MOI of 3) were used as a control. After 48 hours of infection with the viral vector, the cells were washed three times with ice-cold PBS(-), and fixed with cold acetone-methanol (kept at -20°C; the ratio was 1:1 w/w) at -20°C for 20 min. After washing (3 times with PBS at room temperature (RT) for 10 min), the cells were treated with glycine (with PBS/50 mM glycine at RT for 10 min). The cells were washed again (once with PBS at RT for 10 min), and then blocking (10% FBS/10% goat serum/1% BSA/0.05% Tween-20/PBS; at RT for 2 h) was performed. After PBS washes (RT, 2 x 10 min), the primary antibody reaction (RT, 1 h) was carried out using the C terminus-specific anti-human CFTR antibody (10 µg/ml; Chemicon). After PBS washes (RT, 4 x 10 min), the secondary antibody reaction (RT, 30 min) was carried out using an anti-mouse IgG antibody (5 µg/ml, Alexa Fluor 594). After PBS washes ( RT, 4 x 10 min), the cells were observed under a fluorescent microscope and photographed. As shown in Fig. 8, LLC-MK₂ cells infected with SeV carrying the altered hCFTR gene were specifically stained in the fluorescent antibody reaction. A similar result was obtained when BHK-21 and BEAS-2B cells were used.

### Industrial Applicability

The present invention provides a recombinant Sendai virus carrying a human CFTR gene with an altered hypermutable region(s). Sequences that are prone to high frequencies of mutation generation in a foreign gene have been altered in the minus-strand RNA viruses of the present invention. Therefore, the foreign gene can be expressed stably in target tissues. The viruses of the invention can be used as gene therapy vectors for use in *in vivo* and *ex vivo* administration to the body.

## Claims

1. A Sendai virus carrying a human CFTR gene, wherein the gene has been altered at the 5'-AGAAAAAC-3' and 5'-AGAAAAAAC-3' sequence comprised in the sense strand of the wild type of a human CFTR gene in such a way that the amino acid sequence encoded by the 5'-AGAAAAAC-3' and 5'-AGAAAAAAC-3' sequence of the wild-type gene is maintained.

2. A DNA encoding the genome RNA of the Sendai virus of claim 1 or a complementary strand thereof.

3. The Sendai virus of claim 1, wherein the 5'-AGAAAAAC-3' and 5'-AGAAAAAAC-3' sequence is part of a 5'-AGAAAAACTT-3' and 5'-AGAAAAAACTT-3' sequence.

4. A pharmaceutical composition comprising the virus of claim 1 or 3.

5. Use of the virus according to claim 1 or 3 for the preparation of a pharmaceutical composition for gene therapy.

## Patentansprüche

1. Sendai-Virus, das ein menschliches CFTR-Gen trägt, wobei das Gen in der 5'-AGAAAAAC-3'- und 5'-AGAAAAAAC-3'-Sequenz, die im Sensestrang des Wildtyps des menschlichen CFTR-Gens umfasst ist, dergestalt verändert wurde, dass die Aminosäuresequenz, die von der AGAAAAAC-3'- und 5'-AGAAAAAAC-3'-Sequenz des Wildtypgens codiert ist, erhalten bleibt.

2. DNA, die die genomische RNA des Sendai-Virus nach Anspruch 1 codiert, oder ein komplementärer Strang davon.

3. Sendai-Virus nach Anspruch 1, wobei die 5'-AGAAAAAC-3'- und 5'-AGAAAAAAC-3'-Sequenz Teil einer 5'-AGAAAAACTT-3'- und 5'-AGAAAAAACTT-3'-Sequenz ist.

4. Arzneimittel, das das Virus nach Anspruch 1 oder 3 umfasst.

5. Verwendung des Virus nach Anspruch 1 oder 3 für die Herstellung eines Arzneimittels zur Gentherapie.

## Revendications

1. Virus Sendai portant un gène humain CFTR, dans lequel le gène a été altéré au niveau de la séquence 5'-AGAAAAAC-3' et 5'-AGAAAAAAC-3' comprise dans le brin sens du type sauvage d'un gène humain CFTR, de telle sorte que la séquence d'acides aminés codée par la séquence 5'-AGAAAAAC-3' et 5'-AGAAAAAAC-3' du gène de type sauvage est conservée.

2. ADN codant l'ARN génomique du virus Sendai selon la revendication 1 ou un brin complémentaire de celui-ci.

3. Virus Sendai selon la revendication 1, dans lequel la séquence 5'-AGAAAAAC-3' et 5'-AGAAAAAAC-3' fait partie d'une séquence 5'-AGAAAAACTT-3' et 5'-AGAAAAAACTT-3'.

4. Composition pharmaceutique comprenant le virus selon la revendication 1 ou 3.

5. Utilisation du virus selon la revendication 1 ou 3 pour la préparation d'une composition pharmaceutique pour la thérapie génique.
